# FASCICULE DE BREVET EUROPEEN

(11) **EP 0 458 697 B1**
(45) Date de publication et mention de la délivrance du brevet: **27.07.1994**
(21) Numéro de dépôt: 91401312.3
(22) Date de dépôt: 22.05.1991
(51) Int. Cl.: C07D 211/70, C07D 401/06, A61K 31/445

(54) **Trifluorométhylphényltétrahydropyridines N-substituées, procédé pour leur préparation, intermédiaires du procédé et compositions pharmaceutiques les contenant**
N-substituierte Trifluormethylphenyltetrahydropyridine, Verfahren zur Herstellung, Zwischenprodukte, sowie Arzneimittel, die solche enthalten
N-substituted trifluoromethylphenyl tetrahydropyridines, process for their preparation, intermediates and pharmaceutical preparations containing them

(30) Priorité: 23.05.1990 FR 9006474
(43) Date de publication de la demande: 27.11.1991
(73) Titulaire: ELF SANOFI, 75008 Paris (FR); MIDY S.p.A., 20137 Milano (IT)
(72) Inventeur: Guzzi, Umberto, I-20010 Milano (IT); Palmieri, Costantino, I-20137 Milano (IT); Croci, Tiziano, I-20154 Milano (IT)
(74) Mandataire: Gillard, Marie-Louise

(56) Documents cités:
- EP-A- 0 101 381
- GB-A- 2 083 476

## Description

La présente invention concerne de nouvelles 4-(3-trifluorométhylphényl)-1,2,3,6-tétrahydropyridines N-substituées utiles dans le traitement des troubles de la motricité intestinale.

Plus particulièrement, la présente invention concerne de nouveaux composés de formule générale suivante 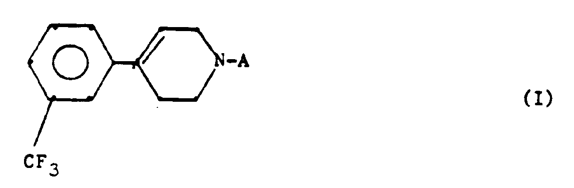
dans laquelle A représente un groupe -E-G ou un groupe -L-M, où
- -E-: est un radical alkylène à chaine droite ou ramifiée contenant de 2 à 4 atomes de carbone,
- -G: représente un groupe choisi parmi naphtyle substitué par un groupe hydroxyle ou (C1-C4)alcoxyle; naphtyle substitué par deux groupes hydroxyle, (C1-C4)alcoxyle ou (C1-C4)alkyle; ou naphtyle substitué par un groupe méthylènedioxy,
- -L-: est un groupe -Q-CH(OH)- dans lequel -Q- est un radical alkylène à chaine droite ou ramifiée contenant de 1 à 3 atomes de carbone, et
- -M: représente un groupe choisi parmi naphtyle; naphtyle substitué par un ou deux groupes hydroxyle, (C1-C4)alcoxyle ou (C1-C4)alkyle ou par un groupe méthylènedioxy; pyridyle; ou pyridyle substitué par un groupe (C1-C4)alkyle,

et leurs sels d'acides minéraux ou organiques.

Le brevet GB-A-881894 décrit des 1-aroylalkyl-4-aryl-1,2,3,6-tétrahydropyridines de formule 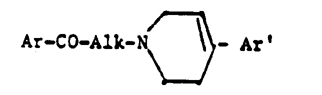
où Ar et Ar′ sont chacun un radical halophényle, alkoxyphényle, diméthoxyphényle, hydroxyphényle, thiényle, trifluorométhylphényle ou alkylphényle et Alk est un radical altylène contenant de 3 à 6 atomes de carbone, ayant une action anticonvulsivante, déprimante du système nerveux central et tranquillisante.

Le brevet BE-837878 décrit, en tant que produits intermédiaires, des tétrahydropyridines de formule 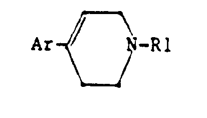
dans laquelle Ar est un groupe aryle éventuellement substitué par un ou plusieurs groupes choisi entre alkyle, alcoxy, halogène et -CF₃ et R1 peut représenter inter alia un groupe aralkyle ou 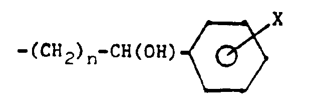
où n est 1, 2, 3 ou 4 et X peut représenter un atome d'hydrogène ou d'halogène.

Le brevet français 1421208 revendique des dérivés tétrahydropyridines de formule 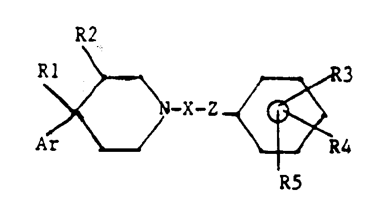
dans laquelle, inter alia, Ar peut représenter un radical phényle, éventuellement substitué par trifluorométhyle; R1 et R2 peuvent représenter, ensemble, une simple liaison; X est un alkylène ou un alcenylène de 2 ou 3 atomes de carbone; Z représente une simple liaison ou un groupe -CH₂-, -CH(OH)-, -CO-, ou -CH(alkyle)- et R3 est un substituant aminé en position méta ou para tandis que R4 et R5 peuvent représenter l'hydrogène ou des groupes substituants.

Ces composés sont décrits comme agents psychotropes, hypothermiques, antiémétiques et potentialisateurs des anesthésiques généraux.

La demande de brevet allemand DE-A-2904826 décrit des tétrahydropyridines de formule 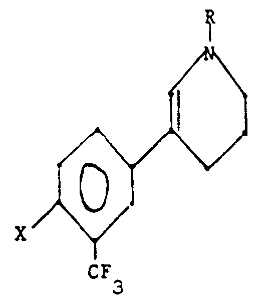
dans laquelle X est un atome d'hydrogène ou de chlore et R est l'hydrogène, un groupe alkyle, alcényle, alcynyle ou phényle-alkyle, qui ont une activité anorexigène et peuvent aussi être utilisées dans le traitement de la dépression.

La demande de brevet européen EP-A-0060176 revendique des 4-(3-trifluorométhylphényl)-1,2,3,6-tétrahydropyridines de formule 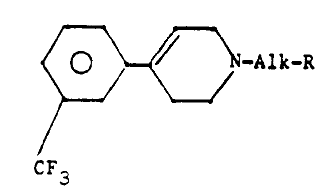
dans laquelle R représente un groupe cyano, acétyle, ou cycloalkyle contenant 3 à 7 atomes de carbone et Alk représente un alkylène à chaine droite ou ramifiée ayant 1 à 4 atomes de carbone, ainsi que leurs sels, à action anorexigène.

Des dérivés 4-aryle-1,2,3,6-tétrahydropyridines de formule 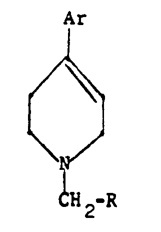
dans laquelle Ar peut être un groupe phényle substitué inter alia par un groupe -CF₃ et R peut représenter un groupe naphtyle, ont été décrits dans la demande de brevet anglais GB 2083476 comme agents psychotropes.

Des dérivés trifluorométhylphényltétrahydropyridines à activité anorexigène de formule 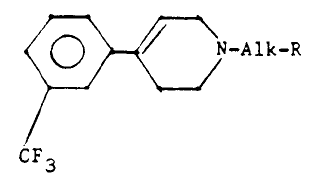
dans laquelle R représente un groupe pyridyle, un groupe pyridyle-3-oxyde, ou un groupe naphtyle, non substitué ou substitué par un groupe alkyle contenant 1 à 4 atomes de carbone et Alk représente un groupe alkylène à chaine droite ou ramifiée contenant 2 à 4 atomes de carbone, ont été décrits dans la demande de brevet européen EP-A-0101381.

Enfin, la demande de brevet international WO-A-8905799 décrit des N-alkényle ou N-alkynyle tétrahydropyridines à structure 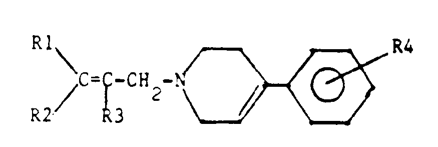
dans laquelle R1, R2 et R3 peuvent être des atomes d'hydrogène ou des groupes alkyle, ou R2 et R3, pris ensemble, peuvent représenter une liaison additionnelle, et R4 est un atome d'halogène, un groupe -CF₃ ou un groupe alkyle, qui possèdent une activité de protection de la muqueuse gastro-intestinale et qui sont donc utiles pour soigner ou prévenir les ulcères gastriques.

On a maintenant trouvé que les trifluorométhylphényltétrahydropyridines N-substituées de formule (I) selon la présente invention augmentent la motricité intestinale chez les mammifères en exerçant donc une activité anti-constipante.

Certains d'entre eux, ont été aussi essayés dans les tests de l'action antidépressive et ils ont montré une activité assez bonne.

Dans le texte de la présente demande les termes "(C1-C4)alkyle" et "(C1-C4)alcoxyle" comprennent des radicaux alkyle et alcoxyle, respectivement, à chaine droite ou ramifiée contenant de 1 à 4 atomes de carbone tels que méthyle, éthyle, propyle, isopropyle, butyle et méthoxyle, éthoxyle, propoxyle, isopropoxyle, butoxyle, les radicaux méthyle et méthoxyle respectivement étant préférés.

Le radical naphtyle substitué peut être un radical 1-naphtyle ou 2-naphtyle portant un ou deux groupes substituants en position 5-, 6-, 7- et 8-.

Les sels des composés de formule (I) selon la présente invention comprennent aussi bien les sels d'addition avec des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels d'addition avec des acides minéraux ou organiques qui permettent une séparation ou une cristallisation convenable des composés de formule (I), tels que le picrate, l'oxalate, etc.

Des composés préférés de la présente invention comprennent les composés de formule (I) dans laquelle A est un groupe -E-G où -E- est un groupe -CH₂-CH₂- dans lequel un des atomes d'hydrogène peut être remplacé par un groupe méthyle et -G est tel que défini ci-dessus.

Des composés particulièrement avantageux de la présente invention comprennent les composés de formule (I) dans laquelle A est un groupe -E-G où -E- est un groupe -CH₂-CH₂- dans lequel un des atomes d'hydrogène peut être remplacé par un groupe méthyle et -G représente un groupe naphtyle substitué par un groupe hydroxy ou méthoxy ou par deux groupes hydroxy, méthoxy ou méthyle.

D'autres composés préférés de la présente invention comprennent les composés de formule (I) dans laquelle A est un groupe -L-M où -L- est un groupe -Q-CH(OH)- dans lequel Q est -CH₂- ou -CH(CH₃)- et M est tel que défini ci-dessus.

Des composés particulièrement avantageux parmi ces derniers sont ceux où -M est un groupe naphtyle, naphtyle substitué par un ou deux groupes hydroxy, méthoxy ou méthyle ou un groupe pyridyle.

Une méthode générale de préparation des composés de formule (I) et de leurs sels comprend la réaction, dans un solvant organique, de la 4-(3-trifluorométhylphényl)-1,2,3,6-tétrahydropyridine (II) 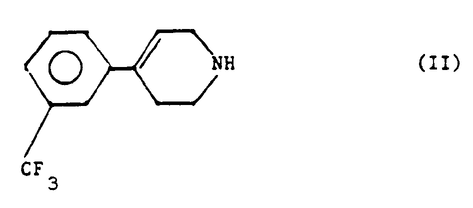
avec un composé de formule (III)

X-A (III)

dans laquelle A a la signification donnée ci-dessus et X représente un atome de chlore, brome, iode, ou un groupe partant , tel que le groupe méthanesulfonyloxy ou p-toluènesulfonyloxy.

Comme solvant organique préférentiél, on utilise un alcanol inférieur tel que le méthanol, l'éthanol, le n-butanol, ou le n-pentanol, mais d'autres solvants tels que l'héxane, le diméthylformamide, le diméthylsulfoxyde, l'acétonitrile, la pyridine et similaires peuvent être utilisés.

La réaction est avantageusement conduite en présence d'une quantité au moins équimoléculaire d'un agent de condensation basique tel qu'un carbonate ou bicarbonate alcalin, comme par exemple le carbonate de sodium ou de potassium, ou une amine tertiaire, comme par exemple la triéthylamine.

La température de réaction peut varier entre la température ambiante et 200°C. En général, après 4 ou 5 heures de chauffage à 80-150°C, la réaction est complète et le produit final ainsi obtenu peut être isolé selon les techniques conventionnelles et il peut éventuellement être transformé dans un de ses sels d'addition avec un acide organique ou inorganique convenablement choisi. La conversion de la base libre dans un sel d'addition est achevée simplement par traitement avec l'acide dans un solvant organique tel qu'un alcanol inférieur comme l'éthanol, ou l'isopropanol, un éther comme le 1,2-diméthoxyéthane ou l'éther isopropylique, un ester, comme l'acétate d'éthyle, ou un hydrocarbure aliphatique ou aromatique comme l'héxane, le benzène ou le toluène.

Lorsqu'on veut préparer des composés de formule (I) dans laquelle A représente un groupe -E-G ou un groupe -L-M, où G, L et M sont tels que définis ci-dessus et -E- est un groupe -R-CH₂- dans lequel -R- est un radical alkylène à chaine droite ou ramifiée contenant de 1 à 3 atomes de carbone, on utilise de préférence une méthode alternative caractérisée en ce qu'on réduit convenablement un composé de formule (IV) 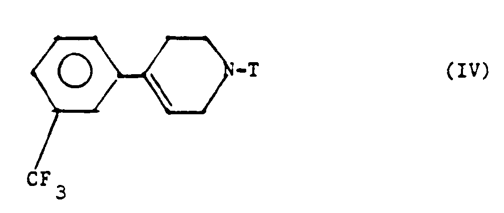
dans laquelle T est un groupe -R-CO-G ou -Q-CO-M où -R-, -Q-, -G et -M sont tels que définis ci-dessus, et si on le désire, on convertit les produits ainsi obtenus en leurs sels d'addition avec des acides.

Si on désire obtenir un composé de formule (I) dans laquelle A représente un groupe -E-G où -E- est un groupe -R-CH₂-, la réduction du groupe carbonyle en groupe méthylène peut être effectuée selon les méthodes connues dans la littérature chimique, telles que, par exemple, la réduction selon Clemmensen, en traitant la cétone (IV) avec un excès de zinc amalgamé dans une solution aqueuse d'acide chlorhydrique à 20-40%, éventuellement en présence d'un solvant organique miscible ou non à l'eau, à la température de reflux du mélange réactionnel, ou en la traitant avec un excès de zinc amalgamé dans un solvant organique contenant de l'acide chlorhydrique gazeux; ou la réduction selon Wolff-Kishner ou Huang-Minlon, qui prévoient la réaction de la cétone (IV) avec de l'hydrazine ou de l'hydrate d'hydrazine et la décomposition de l'hydrazone ainsi obtenue dans un solvant glycolique de point d'ébullition élevé, en la chauffant à 150-200°C en présence de NaOH ou KOH; ou encore la conversion de la cétone (IV) en la toluène-p-sulfonylhydrazone correspondante par réaction avec la toluène-p-sulfonylhydrazine, suivie par la réduction du produit ainsi obtenu par NaBH₄ ou, mieux, NaBH₃CN selon la méthode décrite par R.O.Hutchins et al. dans J. Am. Chem. Soc., 1971, 93, p. 1793 et seq.

Si au contraire, on désire obtenir un composé de formule (I) dans laquelle A est un groupe -L-M, la réduction du groupe carbonyle en groupe alcoolique secondaire peut aisement être effectuée selon des techniques bien connues, en utilisant un hydrure d'aluminium, tel que l'hydrure d' isobutylaluminium (DIBAL), un hydrure d'aluminium et lithium, tel que le LiAlH₄, ou l'hydrure de lithium et trialkoxyaluminium, les hydrures de bore et sodium, et de bore et zinc, les hydrures de lithium et trialkylborane, ou les hydrures similaires qui sont appropriés pour convertir un groupe carbonyle dans un groupe hydroxyméthylène sans altérer les autres groupes fonctionnels de la molécule.

La réaction de réduction est conduite dans un solvant organique inerte convenablement choisi selon l'agent réducteur utilisé. En particulier, en utilisant le DIBAL, les solvants préférentiels sont le benzène, le toluène, ou le 1,2-diméthoxyéthane; en utilisant les hydrures d'aluminium et de lithium, on préfère employer les éthers, tels que l'éther éthylique, le dioxane, le tétrahydrofuranne ou le 1,2-diméthoxyéthane ; en utilisant NaBH₄, on préfère opérer dans du méthanol ou de l'éthanol. La réduction du groupe carbonyle en groupe alcoolique secondaire peut être conduite, selon un mode opérationnel préféré, en utilisant une quantité presque équimoléculaire ou un faible excès du réducteur par rapport au composé (IV) de départ, à une température entre 0°C et la température de reflux du solvant employé, et de préférence à température ambiante ou en dessous et sous atmosphère inerte.

Lorsque la réduction de la cétone (IV) est complète on isole le composé (I) où A représente un groupe -E-G dans lequel -E- est un groupe -R-CH₂-,-G et -R- étant tels que définis ci-dessus, ou un groupe -L-M dans lequel-L- et -M sont tels que définis ci-dessus, sous forme de base libre ou d'un de ses sels.

Les composés de formule (I) dans laquelle A représente un groupe -E-G où -E- est un groupe -CH₂-R-,-R-et-G étant tels que définis ci-dessus, peuvent aussi être préparés par réduction des composés de formule (V) 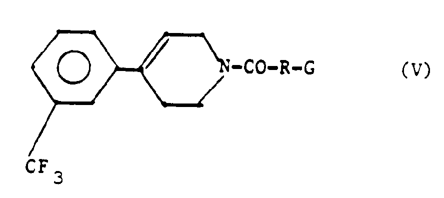
dans laquelle R et G sont tels que définis ci-dessus, suivie éventuellement de la conversion du produit ainsi obtenu en un de ses sels d'addition.

Dans ce cas la réduction du groupe amide peut aisement être conduite en utilisant un hydrure d'aluminium ou un hydrure complexe de lithium et d'aluminium tel que le LiAlH(OCH₃)₃ ou le LiAlH₄ , dans un solvant organique inerte comme les éthers linéaires ou cycliques tels que l'éther diéthylique, le dioxane, le tétrahydrofuranne et le 1,2-diméthoxyéthane, à une température entre 0°C et la température de reflux.

Selon un mode opérationnel préférentiel, on opère avec une quantité équimoléculaire d'hydrure de lithium et d'aluminium par rapport au composé (V) de départ, à température ambiante, dans du tétrahydrofuranne ou de l'éther éthylique et sous atmosphère inerte. Après environ 1 heure la réduction est complète, et on isole le produit de formule (I) dans laquelle A représente un groupe -E-G où -E- est un groupe -CH₂ -R, ainsi obtenu sous forme de base libre ou d'un de ses sels.

Les composés de formule (I) dans laquelle A est un groupe -L-M où -L- est un groupe -Q-CH(OH)- dans lequel -Q- est -CH₂- sont de préférence préparés par réaction du composé de formule (II) avec l'époxyde (VI)

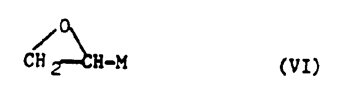

La condensation entre le composé (II) et l'époxyde (VI) est en général achévée en chauffant un mélange des produits de départ dans un alcanol inférieur tel que l'éthanol, l'isopropanol et le butanol, éventuellement en présence d'un agent de condensation basique tel qu'un carbonate alkalin.

Selon un mode opérationnel préférentiel, la condensation est conduite avec une quantité au moins équimoléculaire de l'époxyde (VI) par rapport au composé (II) et en chauffant au reflux le mélange réactionnel pendant quelques heures. Lorsque la réaction est complète, on isole le produit selon les techniques conventionnelles et si l'on désire, on le convertit en un de ses sels d'addition.

Enfin, lorsqu'on obtient par les procédés décrits ci-dessus un composé de formule (I) dans laquelle A est un groupe -E-G ou -L-M où G ou M représentent un groupe naphtyle substitué par un ou deux groupes méthoxy on peut le convertir en le composé correspondant où G ou M représentent un groupe naphtyle substitué par un ou deux groupes hydroxyle par déméthylation, par exemples par traitement du composé méthoxylé avec une solution concentrée d'acide bromhydrique dans l'eau ou dans l'acide acétique.

Un autre objet de la présente invention est donc un procédé pour la préparation d'un composé de formule (I) 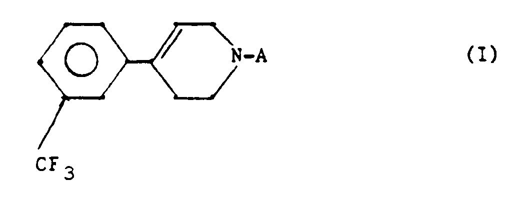
dans laquelle A représente un groupe -E-G ou un groupe -L-M, où
- -E-: est un radical alkylène à chaine droite ou ramifiée contenant de 2 à 4 atomes de carbone,
- -G: représente un groupe choisi parmi naphtyle substitué par un groupe hydroxyle ou (C1-C4)alcoxyle; naphtyle substitué par deux groupes hydroxyle, (C1-C4)alcoxyle ou (C1-C4)alkyle; ou naphtyle substitué par un groupe méthylènedioxy,
- -L: est un groupe -Q-CH(OH)- dans lequel -Q- est un radical alkylène à chaine droite ou ramifiée contenant de 1 à 3 atomes de carbone et
- -M: représente un groupe choisi parmi naphtyle; naphtyle substitué par un ou deux groupes hydroxyle, (C1-C4)alcoxyle ou (C1-C4)alkyle ou par un groupe méthylènedioxy; pyridyle ou pyridyle substitué par un groupe (C1-C4)alkyle,
ou d'un de ses sels d'addition avec des acides minéraux ou organiques, caractérisé en ce que:

(a) on fait réagir la 4-(3-trifluorométhylphényl)-1,2,3,6-tétrahydropyridine de formule (II) 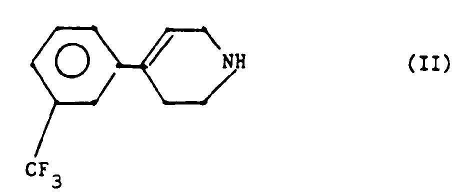 avec un composé de formule (III)

   X-A (III)

   dans laquelle A a la signification donnée ci-dessus et X représente un atome de chlore, brome, iode, ou un groupe partant tel que le groupe méthanesulfonyloxy ou p-toluène-sulfonyloxy; ou
(b) lorsqu'on veut obtenir un composé de formule (I) dans laquelle A représente un groupe -E-G ou -L-M où -E- est un groupe -R-CH₂-, dans lequel -R- est un radical alkylène à chaine droite ou ramifiée contenant de 1 à 3 atomes de carbone, et G, L et M sont tels que défini ci-dessus, on réduit convenablement un composé de formule (IV) 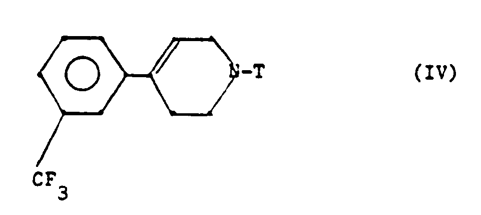 dans laquelle T est un groupe -R-CO-G ou -Q-CO-M où -R-, -Q-, G et M sont tels que définis ci-dessus; ou
(c) lorsqu'on veut obtenir un composé de formule (I) dans laquelle A représente un groupe -E-G où -E- est un groupe -CH₂-R-, -R- et -G étant définis comme ci-dessus, on réduit un composé de formule (V) 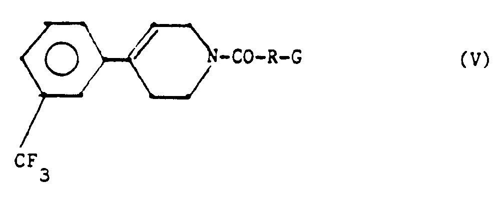 dans laquelle R et G sont tels que définis ci-dessus; ou
(d) lorsqu'on veut obtenir un composé de formule (I) dans laquelle A représente un groupe -L-M où -L- est un groupe -Q-CH(OH)- dans lequel -Q- est -CH₂-, et M est tel que défini ci-dessus, on fait réagir la 4-(3-trifluorométhylphényl)-1,2,3,6-tétrahydropyridine (II) avec un époxyde de formule (VI)

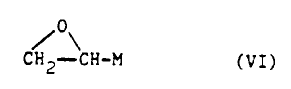

où M est tel que défini ci-dessus;
ce procédé étant ultérieurement caractérisé en ce que,
- lorsqu'on obtient un composé (I) sous forme de base libre, on peut le convertir en un de ses sels d'addition,
- lorsqu'on obtient un composé (I) sous forme de sel d'addition, on peut le convertir en la base libre ou en un autre de ses sels d'addition,
- lorsqu'on obtient un composé de formule (I) dans laquelle A représente un groupe -E-G ou -L-M dans lequel G ou M représentent un groupe naphtyle substitué par un ou deux groupes méthoxyle, on peut le convertir en un composé correspondant où G ou M représentent un groupe naphtyle substitué par un ou deux groupes hydroxyle, par deméthylation.

Les composés de départ de formule (III), lorsqu'ils ne sont pas des produits en commerce peuvent de toute façon être préparés par des méthodes connues. En particulier les composés de formule (III) dans laquelle A représente un groupe -E-G ou -L-M où -E- est un groupe -R-CH₂- et -R-, G, L et M sont tels que définis ci-dessus peuvent être obtenus par acylation de Friedel-Crafts des composés H-G et H-M respectivement dans lesquels G et M sont tels que définis ci-dessus, avec un halogénure d'acide de formule respectivement (VIIa) ou (VIIb) 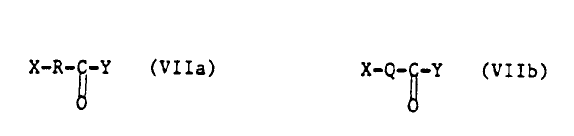
où X,-R et-Q-sont tels que définis ci-dessus et Y est un atome d'halogène tel qu'un atome de chlore ou de brome, en présence d'un acide de Lewis dans les conditions conventionnellement prévues pour cette type de réaction (voir G.A. Olah, Friedel-Crafts and related reactions, volumes 1-5, Interscience Publisher, New York, 1963-1967), suivie par la réduction du groupe carbonyle en groupe méthylène ou hydroxyméthylène.

Plus particulièrement, on peut conduire la réaction de Friedel-Crafts dans un solvant organique inerte tel que le sulfure de carbone, le tétrachlorure de carbone, le chlorure de méthylène, le chlorure d'éthylène, le nitrobenzène et le nitrométhane, en utilisant en tant qu'acide de Lewis du AlCl₃, FeCl₃, SbCl₅, SnCl₄, ZnCl₂, BF₃, TiCl₃, etc., de préférence en quantité équimoléculaire ou en faible excès par rapport à l'agent acylant. En général, on préfère ajouter le catalyseur au mélange de deux réactifs ou ajouter le substrate HG ou HM au mélange du catalyseur et de l'halogénure d'acide (VII). La réaction peut bien être conduite à température ambiante mais, selon les substrats et les réactifs particuliers qu'on utilise, ainsi que les solvants et les catalyseurs qui viennent d'être employés, on peut mieux l'effectuer à une température soit plus basse soit plus élevée que la température ambiante, spécifiquement à une température comprise entre -15°C et la température de reflux du mélange réactionnel, de préférence entre -10°C et 40°C.

Lorsqu'on veut obtenir un composé de formule (III) dans laquelle A est un groupe -E-G ou -L-M où -G ou -M contiennent un ou deux substituants hydroxyle, soit on utilise un catalyseur de Friedel-Crafts qui ne réagit pas avec les groupes hydroxyle tel que le ZnCl₂ ou le BF₃, soit on fait réagir l'halogénure de l'acide (VII) avec les composés HG′ ou HM′ qui correspondent aux composés HG et HM respectivement dans lesquels les groupes hydroxyle ont été protégés sous forme d'éther méthylique et, à la fin de la réaction, on enlève les groupes méthyle simplement par traitement du produit acylé avec une quantité additionnelle de AlCl₃ à chaud.

Selon s'il y a ou non des substituants sur les groupes napthalène, ou pyridine, et leur position éventuelle, la réaction de Friedel-Crafts peut donner lieu à un ou plusieurs isomères de position du composé X-R-CO-G ou X-Q-CO-M. Si on obtient un mélange d'isomères on peut les séparer, par exemple, par chromatographie sur colonne, avant ou après la réaction de réduction du groupe carbonyle.

Pour le passage de réduction du groupe carbonyle, on peut utiliser des techniques connues telles que par exemple celles qui ont été décrites plus haut.

Les composés de formule (III) dans laquelle A représente un groupe -E-G où -E- représente un groupe -CH₂-R- et -R- et -G sont tels que définis ci-dessus peuvent être préparés par conversion de l'acide correspondant (VIII) 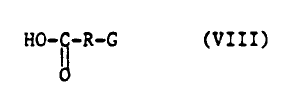
en un ester alkylique inférieur, typiquement l'ester éthylique, réduction du groupe ester en groupe alcoolique primaire par un hydrure mixte tel que le LiAlH4, et déplacement du groupe hydroxyle de la part d'un groupe X par une réaction de substitution nucléophile avec un acide X-H dans laquelle le réactif X- attaque l'atome de carbone polarisé positivement de l'alcool.

A leur tour, les acides de formule (VIII) peuvent être préparés, selon la signification du groupe -R-, par des méthodes différentes qui sont évidentes à l'homme du métier. En particulier par exemple on peut obtenir les acides de formule (VIII) dans laquelle -R- est un groupe -CH₂- en chauffant un mélange du composé G-H et de l'acide chloro- ou bromo-acétique en présence de quantités catalytiques de KBr, pendant quelques heures et isolant le produit désiré par chromatographie.

En alternative et selon un mode opérationnel préféré, on peut préparer les acides (VIII), où -R- est un groupe -CH₂-, par un procédé à trois étapes qui prévoit à la première étape l'acylation de Friedel-Crafts du composé G-H avec le chlorure ou le bromure de l'acide acétique en présence de quantité catalytiques d'un catalyseur de Friedel-Crafts tel que par exemple AlCl₃ ou un autre acide de Lewis, suivie, à la deuxième étape, par la conversion du groupe acétyle en morpholinylthioacétyle, achevée en chauffant à reflux pendant quelques heures un mélange du composé acétylé et d'un faible excès moléculaire de soufre dans la morpholine et, à la troisième étape, par le traitement du composé ainsi obtenu avec de l'hydroxyde de sodium dans de l'éthanol à la température de reflux et l'hydrolyse acide du sel sodique de l'acide (VIII) ainsi obtenu.

On peut convertir les produit ainsi obtenus en dérivés alpha-méthyle-, alpha-éthyle- ou alpha,alpha-diméthyle correspondants (R = -CH(CH₃)-, -CH(CH₂CH₃)-, ou -C(CH₃)₂- respectivement) par alkylation du carbone en position alpha- du carboxyle, effectuée avec de l'hydrure de sodium et une quantité stoechiométrique de iodure de méthyle ou d'éthyle dans le diéthylformamide.

Egalement, on peut convertir les composés de formule (VIII) dans laquelle -R- est un groupe -CH₂- ou -CH(CH₃)- dans les composés correspondants où -R- représente un groupe -CH₂-CH₂- ou -CH₂-CH(CH₃)- respectivement, par la réaction de Arndt-Eistert qui prévoit la conversion du groupe carboxyle dans le chlorure d'acide par traitement avec du chlorure de thionyle, la réaction du chlorure d'acide avec diazométhane et la conversion du composé ainsi obtenu en le produit désiré par traitement avec l'oxyde d'argent (voir F. Arndt et B. Eistert, Berichte, 68, 200 (1935)).

Les composés de formule (III) dans laquelle A représente un groupe -E-G où -E- est un groupe -CH(CH₃)-CH(CH₃)- peuvent être préparés en traitant le composé G-H avec une quantité équimoléculaire d'un halogénure d'alkyle de formule Y-CH(CH₃)-CH(CH₃)-Y où Y est tel que défini ci-dessus en présence de quantités catalytiques d'un acide de Lewis selon la réaction d'alkylation de Friedel-Crafts et, si on desire obtenir un composé de formule (III) dans laquelle X est autre qu'un atome de chlore ou de brome, en déplaçant le groupe Y par un groupe partant.

Dans des cas particuliers on peut utiliser des procédés alternatifs pour la préparation des composés (III), selon les produits de départ qui sont disponibles en commerce. Ces procédés alternatifs peuvent aisement être mis au point par un technicien de laboratoire sur la base de ses connaissances chimiques.

Les composés de départ de formule (IV) peuvent être préparés par réaction de la 4-(3-trifluorométhylphényl)-1,2,3,6-tétrahydropyridine (II) avec un composé de formule X-R-CO-G (IXa) ou X-Q-CO-M (IXb) dans les conditions déjà indiquées pour la réaction entre les composés (II) et (III).

A leur tour les composés de formule (IXa) et (IXb) peuvent être préparés par réaction du composé H-G ou H-M respectivement avec un chlorure d'acide ω-haloalcanoique de formule Y-R-CO-Y ou Y-Q-CO-Y selon la méthodologie de la réaction de Friedel-Crafts, de préférence à température ambiante dans le chlorure de méthylène ou d'éthylène en utilisant AlCl₃ en tant que catalyseur.

A la fin de la réaction on isole le produit desiré, par exemple, par chromatographie, et on le fait réagir avec le composé de formule (II) pour obtenir le composé (IV).

Les composés de formule (IXa) ou (IXb) dans lesquels R ou Q sont un groupe -CH₂- et X est un atome de chlore ou de brome peuvent aussi être obtenus à partir des dérivés acétyles correspondants de formule CH₃-CO-G ou CH₃-CO-M par traitement avec une quantité presque équimoléculaire de brome ou de chlore dans un solvant organique inerte tel que le chloroforme ou le chlorure de méthylène.

Les composés de formule (V) peuvent être obtenus par réaction de la 4-(3-trifluorométhylphényl)-1,2,3,6-tétrahydropyridine (II) avec un dérivé fonctionnel de l'acide de formule (VIII) G-R-CO-OH où -R-et -G sont tels que définis ci-dessus, dans un solvant organique à une température comprise entre -10°C et la température d'ébullition du solvant employé.

Comme dérivé fonctionnel approprié, on peut utiliser l'acide libre activé, l'anhydride, un anhydride mixte, un ester actif ou un halogénure d'acide, le chlorure de préférence. Parmi les esters actifs, l'ester de p-nitrophényle est particulièrement préféré, mais les esters de méthoxyphényle, de trityle, de benzhydryle et similaires sont également convenables.

La température de réaction peut varier entre -10°C et la température d'ébullition du solvant employé, mais en général on opère à la température ambiante ou à 30-50°C. Il est préférable de conduire la réaction au froid lorsqu'elle est exothermique, comme dans le cas où on utilise le chlorure en tant que dérivé fonctionnel de l'acide de formule (VIII).

Comme solvant de réaction, on utilise de préférence un alcool, tel que le méthanol ou l'éthanol, ou un solvant halogéné tel que le chlorure de méthylène, le dichloroéthane, le chloroforme et similaires, mais d'autres solvants organiques compatibles avec les réactifs employés, par exemple le dioxane, le tétrahydrofuranne ou un hydrocarbure tel que l'hexane peuvent être également employés.

La réaction peut être conduite en présence d'un accepteur de protons, par exemple d'un carbonate alcalin ou d'une amine tertiaire, dans le cas où de l'acide chlorhydrique ou un autre acide se libèrerait pendant la réaction.

De préférence on conduit la réaction en partant de l'acide libre en présence d'hexafluorophosphate de benzotriazolyl N-oxytrisdiméthylaminophosphonium (BOP) et d'une amine aliphatique tertiaire.

Enfin, les composés de formule (VI) peuvent être obtenus par cyclisation de la chlorhydrine ou bromhydrine correspondante effectuée en chauffant faiblement une solution de la chloro- ou bromhydrine dans un solvant organique polaire tel qu'un alcanol. La chloro- ou bromhydrine à son tour peut très convenablement être obtenue in situ par reduction de la cétone correspondante par exemple avec du NaBH4 dans un solvant alcoolique tel que le méthanol ou l'éthanol, et être cyclisée directement.

Les composés de départ G-H et M-H sont en général des produits connus. De toute façon ces composés de départ peuvent être préparés par les méthodes décrites dans la littérature pour leur préparation ou même pour la préparation de leurs homologues en y apportant des modifications évidentes.

Les nouveaux composés de départ de formule (IV) et (V) dans lesquelles T, R, G et M sont tels que définis ci-dessus, ainsi que les sels d'addition des composés de formule (IV) avec des acides organiques ou inorganiques, représentent un autre objet spécifique de la présente invention.

On a trouvé que les composés de formule (I), ainsi que leurs sels d'addition pharmaceutiquement acceptables, possèdent une activité procinétique sur l'intestin et qu'ils sont donc utiles pour le traitement des troubles de la motricité intestinale et, en particulier, de la constipation.

Pour l'évaluation de cette activité anticonstipante, les principes actifs représentatifs de la présente invention sont soumis à un test qui prend en considération l'élimination fécale chez le rat.

Des rats mâles Crl : CD(SD)BR (Charles River Italia) pesant entre 220 et 250 g sont placés à huit heures du matin, sans nourriture mais avec libre accès à l'eau, dans des cages individuelles ayant le fond en filet et un plateau situé au-dessous.

A onze heures et demi, les produits sous examen sont administrés par voie orale ou sous-cutanée dans un volume d'eau de 2 ml/kg.

Le schéma de traitement est planifié en utilisant des tableaux "random" et 8 animaux par groupe.

Juste après le traitement, l'ampoule rectale est vidée manuellement des selles éventuellement présentes et les rats sont remis dans leurs cages respectives. Les boulettes sont ramassées, comptées et pesées pour la détermination du poids frais 90 minutes après l'administration du produit par voie sous-cutanée ou bien 210 minutes après l'administration par voie orale. Les selles sont placées dans une étuve et séchées pendant 10 heures à 40°C pour la détermination du poids sec; après 5-6 heures, le poids ne varie plus et les selles contiennent approximativement le même pourcentage d'humidité résiduelle.

Le paramètre utilisé pour le calcul de l'activité est le poids sec en grammes des selles réunies cumulativement pendant les 90 minutes (voie sous-cutanée) ou les 210 minutes (voie orale) qui suivent l'administration du produit. L'analyse statistique des données est effectuée selon le "Duncan's new multiple test".

L'activité relative des composés est calculée sur la base d'un indice d'activité (IA-1g) qui représente la dose active qui stimule l'élimination d'1 g de selles (poids sec); cet indice est calculé en mg/kg sur la droite de régression log-dose effet.

Chez les témoins qui reçoivent seulement le véhicule on n'observe presqu'aucune élimination fécale dans la même période. Chez les animaux traités avec des doses élévées, on peut observer une élimination maximale variable de 12-16 boulettes de selles ayant un poids sec d'environ 1,4 - 1,8 grammes au totale. Au dessus de ces valeurs d'élimination totale on a observé l'apparition d'une évidente diarrhée qui empêche une quantification exacte.

Les composés de formule (I) représentatifs de la classe de composés utilisables pour la préparation des médicaments selon la présente invention ont montré un indice d'activité IA-1g en général plus faible que 15 mg/kg par voie orale que 5 mg/kg par voie sous-cutanée.

Des composés particulièrement avantageux sont les composés des exemples 2, 6, et 7 qui ont montré, sous forme de chlorhydrates correspondants, un indice d'activité IA-1g inférieur à 3 mg/kg par voie orale.

Les composés de formule (I) peuvent être utilisés pour la préparation de médicaments destinés à augmenter la motricité intestinale caractérisés en ce qu'ils contiennent, en tant que principe actif, un ou plusieurs composés de formule générale (I) ou leurs sels avec un support pharmaceutique et éventuellement des produits auxiliaires habituels.

L'administration des composés de formule (I) conformement à l'invention peut être effectuée par voie orale, rectale, sublinguale, transdermique ou par voie parentérale. La voie orale est toutefois préférée et en effet il n'est guère nécessaire de recourir à d'autres moyens d'administration. La quantité de principe actif à administrer pour traiter les troubles de la motricité intestinale depend, comme il est habituel, du composé utilisé, de la nature et de la sévérité de la constipation à traiter, ainsi que du poids des malades et de la voie d'administration.

En général, les doses sont inférieures à environ 70 mg par jour et sont de préférence inférieures à environ 40 mg par jour, lorsque le produit est administré par voie orale. Lorsque le produit est formulé dans une composition pharmaceutique convenable pour l'administraiton parentérale la dose sera comprise entre 0,1 et 25 mg/jour.

Du fait que l'administration doit être adaptée à l'état du patient, une pratique correcte est de commencer avec une dose minimale (de 0,1 à 5 mg, de préférence de 0,5 à 2,5 mg, les doses inférieures étant reservées aux enfants) administrée deux ou trois fois par jour et d'augmenter ensuite la dose ou le nombre d'administrations par jour, jusqu'à observation de l'effet voulu ou à l'apparition d'effets secondaires.

Les compositions pharmaceutiques contenant les composés (I) sont préparées selon les méthodes connues dans la technologie pharmaceutique. En particulier, pour l'administration par voie orale, ces médicaments peuvent se présenter par exemple sous forme de comprimés, de gélules, de capsules, d'elixir, au de sirops.

Lorsqu'on prépare une composition solide sous forme de comprimés, on mélange le principe actif avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation liquide sous forme de sirop ou d'elixir ou pour l'administraiton en gouttes peut contenir l'ingrédient actif conjointement avec un édulcorant, acalorique de préférence, du méthylparaben et du propylparaben comme antiseptiques, ainsi qu'un agent donnant du goût et un colorant approprié, dans un véhicule liquide.

Les poudres ou les granules dispersibles dans l'eau peuvent contenir l'ingrédient actif en mélange avec des agents de dispersion ou des agents mouillants, ou des agents de mise en suspension, tels que la polyvinylpyrrolidone et similaires, de même qu'avec des édulcorants ou des correcteurs du gout.

Le principe actif peut être formulé également sous forme de microcapsules ou de microémulsions, éventuellement avec un ou plusieurs supports ou additifs.

Pour l'administration orale, chaque dose unitaire peut avantageusement contenir de 0,1 à 50 mg de principe actif, de préférence de 0,5 à 10 mg. Dans certains cas, néanmoins, des unités de dosage contenant une quantité plus grande de principe actif peuvent être également envisagées.

Pour l'administration parentérale les compositions pharmaceutiques selon la présente invention contiennent, en plus du principe actif, un ou un mélange de vecteurs, aqueux ou non aqueux, stériles, acceptables du point de vue pharmaceutique.

Ces compositions pharmaceutiques peuvent aussi contenir des adjuvants tels que des agents stabilisants, mouillants, émulsionnants, ou dispersants convenables.

Ces compositions pour l'administration parentérale peuvent être stérilisées, par exemple par filtration à travers un filtre qui retient les bactéries (par exemple, les filtres Millipore R) ou par incorporation d'agents stérilisants dans les compositions.

On peut aussi préparer ces compositions sous forme d'une formulation solide à dissoudre ou suspendre dans l'eau stérile ou dans un autre solvant stérile injectable.

Pour l'administration parentérale, chaque dose unitaire peut avantageusement contenir de 0,05 à 25 mg de principe actif, de préférence de 0,1 à 5 mg.

Les exemples suivants illustrent l'invention sans toutefois la limiter.

### PREPARATION I

### Acide 6,7-diméthoxy-2-naphtylacétique

(a) On chauffe au reflux pendant 4 heures un mélange de 6 g (0,026 moles) de 6,7-diméthoxy-2-acétylnaphtalène, 1 g (0,03 moles) de soufre, 5,5 ml de morpholine et une très faible quantité d'acide p-toluenesulfonique.On refrodit à 70°C et on y ajoute 20 ml d'éthanol. On laisse à repos pendant une nuit, on filtre et on cristallise le produit ainsi isolé dans 100 ml de benzène en obtenant 4,8 g de 4-[(6,7-diméthoxy-2-naphtyl)-thioacétyl]morpholine. P.f. 158-161°C.
(b) On chauffe au reflux pendant 4 heures un mélange de 4,7 g (0,014 moles) du produit obtenu ci-dessus, 2,3 g (0,057 moles) de NaOH, 50 ml d'éthanol et 23 ml d'eau, on concentre à sec et on dissout le résidu dans l'eau. On lave la solution aqueuse à l'éther éthylique, on y ajoute de l'acide chlorhydrique concentré jusqu'à un pH d'environ 1 et on laisse cristalliser. On obtient 2,3 g du produit indiqué dans le titre. P.f. 134-136°C.

### PREPARATION II

### (6,7-diméthoxy-napht-2-yl)oxirane

(a) On ajoute goutte à goutte une solution de 4,5 g (0,029 moles) de brome dans 30 ml de chloroforme à une solution de 6,7 g (0,029 moles) de 6,7-diméthoxy-2-acétylnaphtalène dans 70 ml de chloroforme et 15 ml de méthanol. Dès que le mélange réactionnel s'est décoloré, on concentre à sec et on solidifie le résidu en le traitant avec un mélange éther éthylique/éther isopropylique. Le produit ainsi obtenu est trituré dans du méthanol et donne 6 g de 6,7-diméthoxy-2-(2-bromoacétyl)naphtalène. P.f. 120-122°C.
(b) On ajoute par portions 1,6 g de NaBH4 à une suspension de 6 g (0,019 moles) du produit obtenu ci-dessus dans 80 ml de méthanol. La température du mélange réactionnel s'élève à 40°C et on obtient une solution. On agite pendant 1 heure, on concentre à sec et on dissout le résidu ainsi obtenu dans de l'acétate d'éthyle. On lave la solution organique à l'eau, on la sèche et concentre à sec. On obtient un résidu qui est trituré dans de l'éther isopropylique et donne 2,6 g du produit indiqué dans le titre. P.f. 115-117°C.

### Exemple 1

### Chlorhydrate de 1-[4-(3-trifluorométhylphényl)-1,2,3,6-tétrahydropyrid-1-yl]-2-(6-méthoxy-napht-2-yl)propane

(a) On agite à la température ambiante pendant 4 heures une solution de 3,5 g (0,015 moles) de l'acide 6-méthoxy-alpha-méthyl-2-naphtylacétique, 4,3 ml (0,03 moles) de triéthylamine, 3,9 g (0,015 moles) de chlorhydrate de 4-(3-trifluorométhylphényl)-1,2,3,6-tétrahydropyridine , 6 g (0,015 moles) de hexafluorophosphate de benzotriazolyl-N-oxytris-(diméthylamino)phosphonium (BOP) (0,015 moles) dans 40 ml de chlorure de méthylène. On y ajoute 150 ml d'acétate d'éthyle et après on lave successivement à l'eau,avec une solution à 10% de NaHCO3, à l'eau,avec solution 1N d'acide chlorhydrique et enfin à l'eau. On filtre sur silice et on concentre à sec. On obtient 4 g de 1-[4-(3-trifluorométhylphényl)-1,2,3,6-tétrahydropyrid-1-yl]-1-oxo-2-(6-méthoxy-napht-2-yl)propane sous forme d'une huile épaisse.
(b) On ajoute goutte à goutte une solution du produit ainsi obtenu dans 40 ml de tétrahydrofuranne dans une suspension de 0,7 g de LiAlH4 (0,018 moles) dans 10 ml de tétrahydrofuranne et on agite le mélange réactionnel pendant 3 heures. On ajoute très lentement 1,5 g d'eau et on laisse sous agitation pendant 2 heures supplémentaires. On filtre, on concentre à sec, et on dissout le résidu ainsi obtenu dans 80 ml d'acétate d'éthyle. On filtre sur silice et on concentre à sec le filtrat. On dissout le résidu dans de l'acétone et on y ajoute de l'acide chlorhydrique gazeux. Le chlorhydrate qui précipite est séparé par filtration et cristallisé dans 40 ml d'acétone en donnant 1,5 g de chlorhydrate de 1-[4-(3-trifluorométhylphényl)-1,2,3,6-tétrahydropyrid-1-yl]-2-(6-méthoxy-napht-2-yl)propane. P.f. 182-184°C.

### Exemple 2

### Chlorhydrate de 2-[4-(3-trifluorométhylphényl)-1,2,3,6-tétrahydropyrid-1-yl]-1-(napht-2-yl)éthanol

On chauffe au reflux pendant 2 heures un mélange de 2,7 g (0,01 mole) de 4-(3-trifluorométhylphényl)-1,2,3,6-tétrahydropyridine, 2,7 g (0,02 moles) de carbonate de potassium et 2,7 g (0,015 moles) de 2-naphtyl-oxirane dans 50 ml de butanol. On concentre à sec, on dissout le résidu dans de l'acétate d'éthyle, on lave la solution à l'eau, on la sèche et on concentre à sec. On triture le résidu dans de l'éther éthylique, on filtre et on dissout le produit ainsi isolé dans de l'isopropanol chaud, on y ajoute de l'acide chlorhydrique concentré et on laisse cristalliser. On obtient ainsi 1,9 g du produit du titre. P.f. 245-247°C.

### Exemple 3

### Chlorhydrate de 1-[4-(3-trifluorométhylphényl) -1,2,3,6-tétrahydropyrid-1-yl]-2-(6-méthoxy-napht-2-yl)éthane

(a) On agite à la température ambiante pendant 5 heures une solution de 1,8 g (0,009 moles) de l'acide 6-méthoxy-2-naphtyl)-acétique, 2,4 g (0,009 moles) de chlorhydrate de 4-(3-trifluorométhylphényl)- 1,2,3,6-tétrahydropyridine, 2,4 ml (0,018 moles) de triéthylamine, 3,7 g (0,009 moles) de BOP et 40 ml de chlorure de méthylène, et après on la concentre à sec. On dissout le résidu dans un mélange éther éthylique/eau, on sépare la phase organique, on la lave successivement avec une solution d'HCl 1N, à l'eau,avec une solution 5% de NaOH, et ensuite à l'eau. On filtre sur silice et on concentre le filtrat à sec. On obtient 3,1 g de 1-[4-(3-trifluorométhylphényl)-1,2,3,6-tétrahydropyrid-1-yl]-1-oxo-2-(6-méthoxy-napht-2-yl)éthane. P.f. 102-105°C.
(b) On ajoute goutte à goutte une solution de 3 g (0,007 moles) du produit obtenu ci-dessus dans 30 ml de tétrahydrofuranne à une suspension de 0,5 g (0,013 moles) de LiAlH4 dans 30 ml d'éther éthylique et on agite le mélange réactionnel à température ambiante pendant 3 heures. On y ajoute 1,2 g d'eau, on agite, toujours à température ambiante, pendant 2 heures. On filtre et on concentre le filtrat à sec. On dissout le résidu dans de l'acétate d'éthyle, on lave à l'eau, on sèche et on y ajoute de l'isopropanol saturé d'acide chlorhydrique gazeux. On sépare le précipité par filtration et on le cristallise dans 150 ml d'éthanol 95% en obtenant 2 g du produit indiqué dans le titre. P.f. 260-263°C.

### Exemple 4

### Bromhydrate de 1-[4-(3-trifluorométhylphényl) -1,2,3,6-tétrahydropyrid-1-yl]-2-(6-hydroxy-napht-2-yl)éthane

On chauffe au reflux pendant 4 heures un mélange de 0,6 g (0,0013 moles) de chlorhydrate de 1-[-4-(3-trifluorométhylphényl)-1,2,3,6-tétrahydropyrid-1-yl]-2-(6-méthoxy-napht-2-yl)éthane (obtenu comme décrit dans l'Exemple 3) et 20 ml d'une solution à 33% d'acide bromhydrique dans l'acide acétique. On isole par filtration le précipité et on le cristallise dans de l'éthanol 95% en obtenant 0,3 g du produit indiqué dans le titre. P.f. 210-212°C.

### Exemple 5

### Chlorhydrate de 1-[4-(3-trifluorométhylphényl) -1,2,3,6-tétrahydropyrid-1-yl]-2-(6,7-diméthyl-napht-2-yl)éthane

(a) On agite à la température ambiante pendant 4 heures une solution de 1 g (0,0046 moles) d'acide 6,7-diméthyl-2-naphtyl-acétique, 1,2 g (0,0046 moles) de chlorhydrate de 4-(3-trifluorométhylphényl)- 1,2,3,6-tétrahydropyridine, 1,3 ml (0,009 moles) de triéthylamine, 1,8 g (0,0046 moles) de BOP et 30 ml de chlorure de méthylène. On concentre à sec, on dissout le résidu dans de l'éther éthylique et on lave la solution organique, successivement, à l'eau, avec une solution de NaOH 1N, à l'eau, avec une solution de HCl 1N et enfin à l'eau. On filtre sur silice et on concentre le filtrat à sec. On obtient 0,6 g de 1-[4-(3-trifluorométhylphényl)-1,2,3,6-tétrahydropyrid-1-yl]-1-oxo-2-(6,7-diméthyl-2-naphtyl)éthane.
(b) On agite à température ambiante pendant 3 heures une suspension de 0,6 g (0,0014 moles) du produit obtenu ci-dessus, 0,25 g (0,006 moles) de LiAlH4 dans 10 ml d'éther éthylique et 10 ml de tétrahydrofuranne. On y ajoute 0,7 g d'eau, on agite à température ambiante pendant 2 heures, on filtre et on concentre le filtrat à sec. On dissout le résidu ainsi obtenu dans de l'isopropanol chaud, on acidifie la solution par de l'acide chlorhydrique dans l'éthanol et on laisse cristalliser. On obtient 0,2 g du produit indiqué dans le titre. P.f. 245-247°C.

### Exemple 6

### Chlorhydrate de 1-[4-(3-trifluorométhylphényl) -1,2,3,6-tétrahydropyrid-1-yl]-2-(6,7-diméthoxy-napht-2-yl)éthane

(a) On agite à température ambiante pendant 4 heures un mélange de 2,3 g (0,0093 moles) d'acide 6,7-diméthoxy-2-naphtylacétique, 2,4 g (0,009 moles) de chlorhydrate de 4-(3-trifluorométhylphényl)-1,2,3,6-tétrahydropyridine, 2,5 ml (0,018 moles) de triéthylamine et 3,8 g (0,009 moles) de BOP et 50 ml de chlorure de méthylène. On concentre à sec, on dissout le résidu dans un mélange éther éthylique/eau et on sépare la phase organique. On la lave, successivement avec une solution IN d'acide chlorhydrique, à l'eau, avec une solution 1N d'hydroxyde de sodium et à l'eau. On sèche et on concentre à sec. On purifie le résidu ainsi obtenu par chromatographie en éluant avec un mélange éther éthylique/acétate d'éthyle 1/1. On obtient ainsi 2,6 g de 1-[4-(3-trifluorométhylphényl)-1,2,3,6-tétrahydropyrid-1-yl]-1-oxo-2-(6,7-diméthoxy-2-naphtyl)éthane.
(b) On ajoute goutte à goutte une solution de 2,6 g (0,0057 moles) du produit obtenu ci-dessus dans 40 ml d'éther éthylique à une suspension de 0,5 g (0,013 moles) de LiAlH₄ dans 20 ml d'éther éthylique et on agite le mélange réactionnel ainsi obtenu à température ambiante pendant 4 heures. On détruit l'excès de LiAlH₄ en y ajoutant avec précaution 1,2 ml d'eau. On laisse au repos pendant une nuit, on filtre et on lave la phase organique avec une solution 1N d'hydroxyde de sodium et après à l'eau. On sèche, on concentre à sec, on dissout le résidu dans de l'isopropanol, on rend la solution acide par addition d'acide chlorhydrique et on laisse cristalliser en obtenant 1,5 g du produit indiqué dans le titre qui est cristallisé de nouveau dans de l'éthanol. P.f. 215-217°C.

### Exemple 7

### Chlorhydrate de 1-[4-(3-trifluorométhylphényl) -1,2,3,6-tétrahydropyrid-1-yl]-2-hydroxy-2-(6,7-diméthoxy-napht-2-yl)éthane

On chauffe au reflux pendant 4 heures une solution de 2,5 g (0,014 moles) de 1-(6,7-diméthoxynapht-2-yl)-1,2-époxyéthane, 3,1 g (0,013 moles) de 4-(3-trifluorométhylphényl)-1,2,3,6-tétrahydropyridine et 50 ml d'éthanol. On laisse au repos pendant une nuit et on filtre. On dissout le produit ainsi isolé dans un solution bouillante de 20 ml d'éthanol et 2 ml d'acide chlorhydrique à 37% et on laisse cristalliser en obtenant 1,5 g du produit indiqué dans le titre. P.f. 232-234°C.

### Exemple 8

### Chlorhydrate de 1-[4-(3-trifluorométhylphényl) -1,2,3,6-tétrahydropyrid-1-yl]-2-hydroxy-2-(pyrid-2-yl)éthane

On chauffe au reflux pendant 4 heures 2,4 g (0,01 moles) de 4-(3-trifluorométhylphényl)-1,2,3,6-tétrahydropyridine et 1,21 g (0,01 moles) de 2-pyridyloxirane (JACS, 1976, 98 (7), 1952) dans 45 ml d'éthanol absolu. On évapore à sec et on purifie le résidu par chromatographie sur colonne de gel de silice en éluant par un mélange acétate d'éthyle/méthanol 9/1. On réunit les fractions qui contiennent le produit, on les évapore à sec et on reprend le résidu dans de l'acétone. On j' ajoute une solution d'isopropanol saturé d'acide chlorhydrique gazeux, on sépare le précipité et on le cristallise dans 140 ml d'isopropanol. On obtient 1,5 g du produit indiqué dans le titre. P.f. 192-196°C.

## Revendications (Revendications pour l'(les) Etat(s) contractant(s) suivant(s): AT, BE, CH, DE, DK, FR, GB, IT, LI, LU, NL, SE)

1. Une 4-(3-trifluorométhylphényl)-1,2,3,6-tétrahydropyridine N-substituée de formule 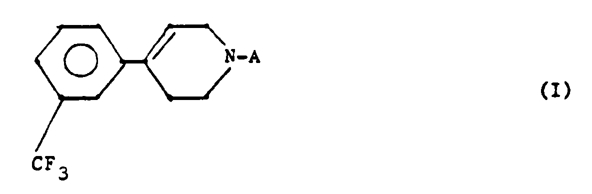 dans laquelle A représente un groupe -E-G ou un groupe -L-M, où
-E- est un radical alkylène à chaine droite ou ramifiée contenant de 2 à 4 atomes de carbone,
-G représente un groupe choisi parmi naphtyle substitué par un groupe hydroxyle ou (C1-C4)alcoxyle; naphtyle substitué par deux groupes hydroxyle, (C1-C4)alcoxyle ou (C1-C4)alkyle; ou naphtyle substitué par un groupe méthylènedioxy,
-L- est un groupe -Q-CH(OH)- dans lequel -Q- est un radical alkylène à chaine droite ou ramifiée contenant de 1 à 3 atomes de carbone, et
-M représente un groupe choisi parmi naphtyle; naphtyle substitué par un ou deux groupes hydroxyle, (C1-C4)alcoxyle ou (C1-C4)alkyle ou par un groupe méthylènedioxy; pyridyle ou pyridyle substitué par un groupe (C1-C4)alkyle,
et leurs sels d'acides minéraux ou organiques.

2. Une 4-(3-trifluorométhylphényl)-1,2,3,6-tétrahydropyridine de la revendication 1 caractérisée en ce que A est un groupe -E-G où -E- est un groupe -CH₂-CH₂- dans lequel un des atomes d'hydrogène peut être remplacé par un groupe méthyle et -G est tel que défini dans la revendication 1.

3. Une 4-(3-trifluorométhylphényl)-1,2,3,6-tétrahydropyridine de la revendication 2 caractérisée en ce que -G représente un groupe naphtyle substitué par un groupe hydroxy ou méthoxy ou par deux groupes hydroxy, méthoxy ou méthyle.

4. Une 4-(3-trifluorométhylphényl)-1,2,3,6-tétrahydropyridine de la revendication 1 caractérisée en ce que A est un groupe -L-M où -L- représente un groupe -Q-CH(OH)- dans lequel -Q- est -CH₂- ou -CH(CH₃)- et -M est tel que défini dans la revendication 1.

5. Une 4-(3-trifluorométhylphényl)-1,2,3,6-tétrahydropyridine de la revendication 4 caractérisée en ce que -M représente un groupe naphtyle; naphtyle substitué par un groupe ou deux groupes hydroxy, méthoxy, méthyle; ou un groupe pyridyle.

6. Un procédé de préparation d'un composé de formule (I) 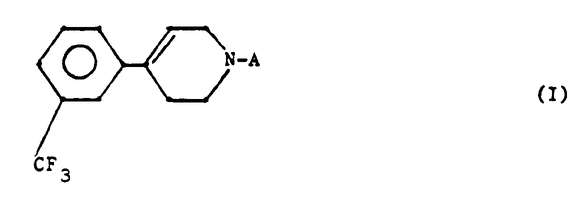 dans laquelle A représente un groupe -E-G ou un groupe -L-M, où
-E- est un radical alkylène à chaine droite ou ramifiée contenant de 2 à 4 atomes de carbone,
-G représente un groupe choisi parmi naphtyle substitué par un groupe hydroxyle ou (C1-C4)alcoxyle; naphtyle substitué par deux groupes hydroxyle, (C1-C4)alcoxyle ou (C1-C4)alkyle; ou naphtyle substitué par un groupe méthylènedioxy,
-L est un groupe -Q-CH(OH)- dans lequel -Q- est un radical alkylène à chaine droite ou ramifiée contenant de 1 à 3 atomes de carbone et
-M représente un groupe choisi parmi naphtyle; naphtyle substitué par un ou deux groupes hydroxyle, (C1-C4)alcoxyle ou (C1-C4)alkyle ou par un groupe méthylènedioxy; pyridyle ou pyridyle substitué par un groupe (C1-C4)alkyle,
ou d'un de ses sels d'addition avec des acides minéraux ou organiques, caractérisé en ce que:
(a) on fait réagir la 4-(3-trifluorométhylphényl)-1,2,3,6-tétrahydropyridine (II) 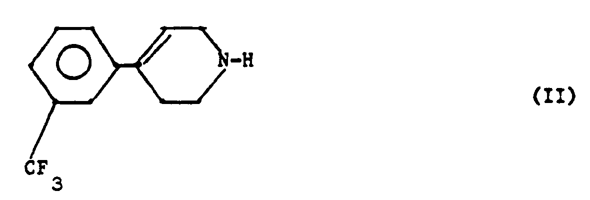 avec un composé de formule (III)
X-A (III)
dans laquelle A a la même signification donnée ci-dessus et X représente un atome de chlore, brome, iode, ou un groupe partant ; ou
(b) lorsqu'on veut obtenir un composé de formule (I) dans laquelle A représente un groupe -E-G ou -L-M où -E- est un groupe -R-CH₂-, dans lequel -R- est un radical alkylène à chaine droite ou ramifiée contenant de 1 à 3 atomes de carbone et G, L et M sont tels que défini ci-dessus, on réduit convenablement un composé de formule (IV) 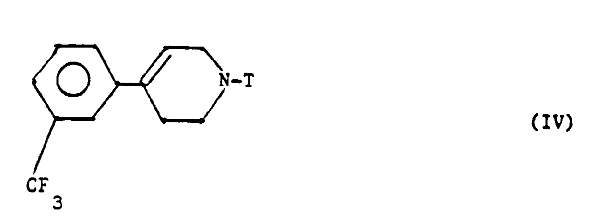 dans laquelle T est un groupe -R-CO-G ou -Q-CO-M où -R-, -Q-, G et M sont tels que définis ci-dessus; ou
(c) lorsqu'on veut obtenir un composé de formule (I) dans laquelle A représente un groupe -E-G où -E- est un groupe -CH₂-R-, -R- et -G étant définis comme ci-dessus, on réduit un composé de formule (V) 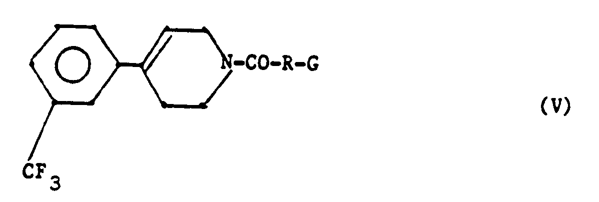 dans laquelle R et G sont tels que définis ci-dessus; ou
(d) lorsqu'on veut obtenir un composé de formule (I) dans laquelle A représente un groupe -L-M où -L- est un groupe -Q-CH(OH)- dans lequel -Q- est -CH₂-, et M est tel que défini ci-dessus, on fait réagir la 4-(3-trifluorométhylphényl)-1,2,3,6-tétrahydropyridine (II) avec un époxyde de formule (VI) 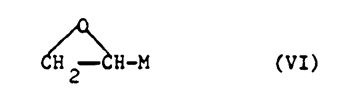
où M est tel que défini ci-dessus; ce procédé étant ultérieurement caractérisé en ce que,
- lorsqu'on obtient un composé (I) sous forme de base libre, on peut le convertir en un de ses sels d'addition,
- lorsqu'on obtient un composé (I) sous forme de sel d'addition, on peut le convertir en la base libre ou en un autre de ses sels d'addition,
- lorsqu'on obtient un composé de formule (I) dans laquelle A représente un groupe -E-G ou -L-M dans lequel G ou M représentent un groupe naphtyle substitué par un ou deux groupes méthoxyle, on peut le convertir en le composé correspondant où -G ou M représentent un groupe naphtyle substitué par un ou deux groupes hydroxyle, par deméthylation.

7. Un composé de formule (IV) 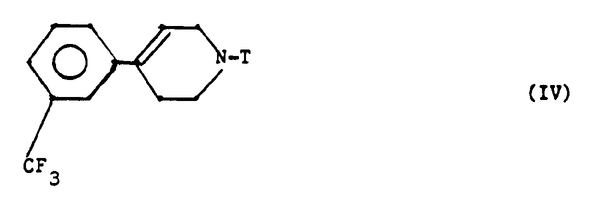 dans laquelle T est un groupe -R-CO-G ou -Q-CO-M où -R- et -Q-, sont un radical alkylène à chaine droite ou ramifiée contenant de 1 à 3 atomes de carbone, -G représente un groupe choisi parmi naphtyle substitué par un groupe hydroxyle ou (C1-C4)alcoxyle; naphtyle substitué par deux groupes hydroxyle, (C -C4)alcoxyle ou (C1-C4)alkyle; ou naphtyle substitué par un groupe méthylènedioxy et -M représente un groupe choisi entre naphtyle; naphtyle substitué par un ou deux groupes hydroxyle, (C1-C4)alcoxyle ou (C1-C4)alkyle ou par un groupe méthylènedioxy; pyridyle; ou pyridyle substitué par un groupe (C1-C4)alkyle et leurs sels d'addition avec des acides mineraux ou organiques.

8. Un composé de formule (V) 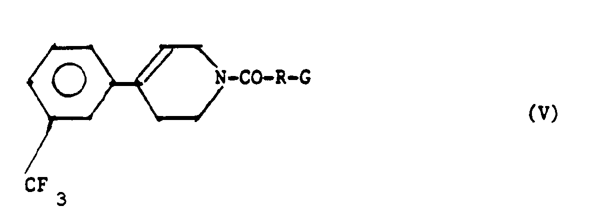 dans laquelle -R- est un radical alkylène à chaine droite ou ramifiée contenant de 1 à 3 atomes de carbone, et -G représente un groupe choisi parmi naphtyle substitué par un groupe hydroxyle ou (C1-C4)alcoxyle; naphtyle substitué par deux groupes hydroxyle, (C1-C4)alcoxyle ou (C1-C4)alkyle; ou naphtyle substitué par un groupe méthylènedioxy.

9. Une composition pharmaceutique caracterisée en ce qu'elle contient à titre d'ingredient actif au moins un composé de formule (I) 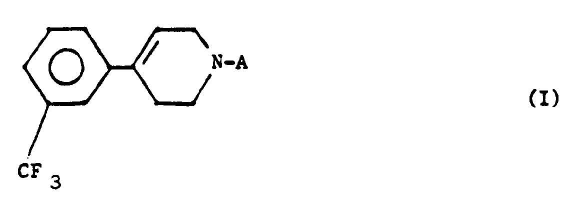 dans laquelle A représente un groupe -E-G ou un groupe -L-M, où
-E- est un radical alkylène à chaine droite ou ramifiée contenant de 2 à 4 atomes de carbone,
-G représente un groupe choisi parmi naphtyle substitué par un groupe hydroxyle ou (C1-C4)alcoxyle; naphtyle substitué par deux groupes hydroxyle, (C1-C4)alcoxyle ou (C1-C4)alkyle; ou naphtyle substitué par un groupe méthylènedioxy,
-L- est un groupe -Q-CH(OH)- dans lequel -Q- est un radical alkylène à chaine droite ou ramifiée contenant de 1 à 3 atomes de carbone, et
-M représente un groupe choisi parmi naphtyle; naphtyle substitué par un ou deux groupes hydroxyle, (C1-C4)alcoxyle ou (C1-C4)alkyle ou par un groupe méthylènedioxy; pyridyle; ou pyridyle substitué par un groupe (C1-C4)alkyle,
ou un de ses sels d'addition pharmaceutiquement acceptables,avec un véhicule et/ou des excipients pharmaceutiquement acceptables.

10. Une composition pharmaceutique de la revendication 9 pour l'utilisation dans le traitement des troubles de la motricité intestinale.

## Revendications (Revendications pour l'(les) Etat(s) contractant(s) suivant(s): ES)

1. Procédé de préparation d'un composé de formule (I) 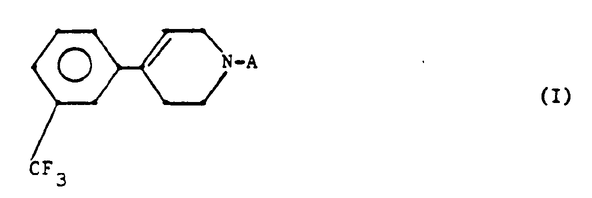 dans laquelle A représente un groupe -E-G ou un groupe -L-M, où
-E- est un radical alkylène à chaine droite ou ramifiée contenant de 2 à 4 atomes de carbone,
-G représente un groupe choisi parmi naphtyle substitué par un groupe hydroxyle ou (C1-C4)alcoxyle; naphtyle substitué par deux groupes hydroxyle, (C1-C4)alcoxyle ou (C1-C4)alkyle; ou naphtyle substitué par un groupe méthylènedioxy,
-L est un groupe -Q-CH(OH)- dans lequel -Q- est un radical alkylène à chaine droite ou ramifiée contenant de 1 à 3 atomes de carbone et
-M représente un groupe choisi parmi naphtyle; naphtyle substitué par un ou deux groupes hydroxyle, (C1-C4)alcoxyle ou (C1-C4)alkyle ou par un groupe méthylènedioxy; pyridyle ou pyridyle substitué par un groupe (C1-C4)alkyle,
ou d'un de ses sels d'addition avec des acides minéraux ou organiques, caractérisé en ce que:
(a) on fait réagir la 4-(3-trifluorométhylphényl)-1,2,3,6-tétrahydropyridine (II) 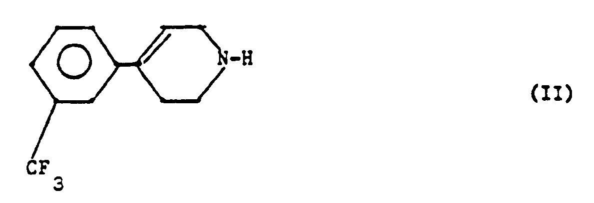 avec un composé de formule (III)
X-A (III)
dans laquelle A a la même signification donnée ci-dessus et X représente un atome de chlore, brome, iode, ou un groupe partant; ou
(b) lorsqu'on veut obtenir un composé de formule (I) dans laquelle A représente un groupe -E-G ou -L-M où -E- est un groupe -R-CH₂-, dans lequel -R- est un radical alkylène à chaine droite ou ramifiée contenant de 1 à 3 atomes de carbone et G, L et M sont tels que défini ci-dessus, on réduit convenablement un composé de formule (IV) 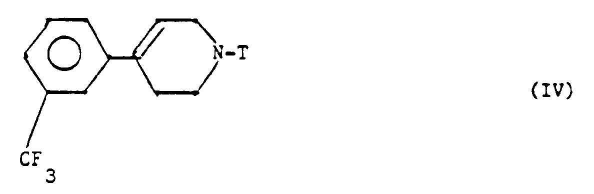 dans laquelle T est un groupe -R-CO-G ou -Q-CO-M où -R-, -Q-, G et M sont tels que définis ci-dessus; ou
(c) lorsqu'on veut obtenir un composé de formule (I) dans laquelle A représente un groupe -E-G où -E- est un groupe -CH₂-R-, -R- et -G étant définis comme ci-dessus, on réduit un composé de formule (V) 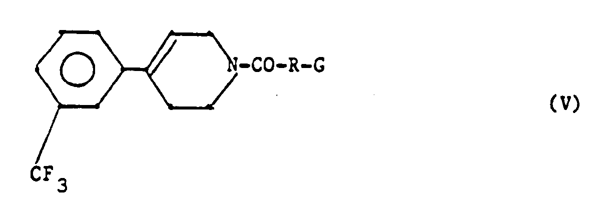 dans laquelle R et G sont tels que définis ci-dessus; ou
(d) lorsqu'on veut obtenir un composé de formule (I) dans laquelle A représente un groupe -L-M où -L- est un groupe -Q-CH(OH)- dans lequel -Q- est -CH₂-, et M est tel que défini ci-dessus, on fait réagir la 4-(3-trifluorométhylphényl)-1,2,3,6-tétrahydropyridine (II) avec un époxyde de formule (VI) 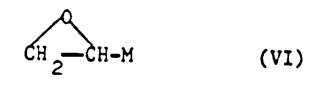 où M est tel que défini ci-dessus;
ce procédé étant ultérieurement caractérisé en ce que,
- lorsqu'on obtient un composé (I) sous forme de base libre, on peut le convertir en un de ses sels d'addition,
- lorsqu'on obtient un composé (I) sous forme de sel d'addition, on peut le convertir en la base libre ou en un autre de ses sels d'addition,
- lorsqu'on obtient un composé de formule (I) dans laquelle A représente un groupe -E-G ou -L-M dans lequel C ou M représentent un groupe naphtyle substitué par un ou deux groupes méthoxyle, on peut le convertir en le composé correspondant où -G ou M représentent un groupe naphtyle substitué par un ou deux groupes hydroxyle, par deméthylation.

2. Procédé selon la revendication 1 pour la préparation d'une 4-(3-trifluorométhylphényl)-1,2,3,6-tétrahydropyridine de formule (I) dans laquelle A est un groupe -E-G où -E- est un groupe -CH₂-CH₂- où un des atomes d'hydrogène peut être remplacé par un groupe méthyle et -G est tel que défini dans la revendication 1.

3. Procédé selon la revendication 1 pour la préparation d'une 4-(3-trifluorométhylphényl-1,2,3,6-tétrahydropyridine de formule (I) dans laquelle A est un groupe -E-G où -E- est un groupe -CH₂-CH₂- où un des atomes d'hydrogène peut être remplacé par un groupe méthyle et -G représente un groupe naphtyle substitué par un groupe hydroxy ou méthoxy ou par deux groupes hydroxy, méthoxy ou méthyle.

4. Procédé selon la revendication 1 pour la préparation d'une 4-(3-trifluorométhylphényl)-1,2,3,6-tétrahydropyridine de formule (I) dans laquelle A est un groupe -L-M où -L- représente un groupe -Q-CH(OH)- dans lequel -Q- est CH₂- ou CH(CH₃)- et -M est tel que défini dans la revendication 1.

5. Procédé selon la revendication 1 pour la préparation d'une 4-(3-trifluorométhylphényl)-1,2,3,6-tétrahydropyridine de formule (I) dans laquelle A est un groupe -L-M où -L- représente un groupe -Q-CH(OH)- dans lequel -Q- est -CH₂- ou -CH(CH₃)- et -M représente un groupe naphtyle ; naphtyle substitué par un groupe ou deux groupes hydroxy, méthoxy, méthyle ; ou un groupe pyridyle.

6. Procédé de préparation d'un composé de formule (IV) 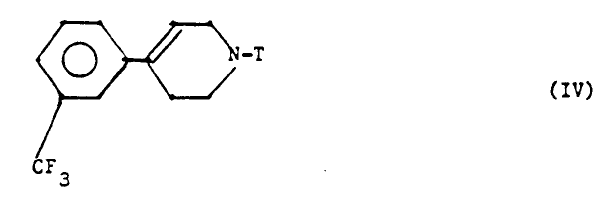 dans laquelle T est un groupe -R-CO-G ou -Q-CO-M où -R- et -Q-, sont un radical alkylène à chaine droite ou ramifiée contenant de 1 à 3 atomes de carbone, -G représente un groupe choisi parmi naphtyle substitué par un groupe hydroxyle ou (C1-C4)alcoxyle; naphtyle substitué par deux groupes hydroxyle, (C -C4)alcoxyle ou (C1-C4)alkyle; ou naphtyle substitué par un groupe méthylènedioxy et -M représente un groupe choisi parmi naphtyle; naphtyle substitué par un ou deux groupes hydroxyle, (C1-C4)alcoxyle ou (C1-C4)alkyle ou par un groupe méthylènedioxy; pyridyle; ou pyridyle substitué par un groupe (C1-C4)alkyle et de ses sels d'addition avec des acides minéraux ou organiques, caractérisé en ce qu'on fait réagir la 4-(3-trifluorométhylphényl)-1,2,3,6-tétrahydropyridine de formule (II) 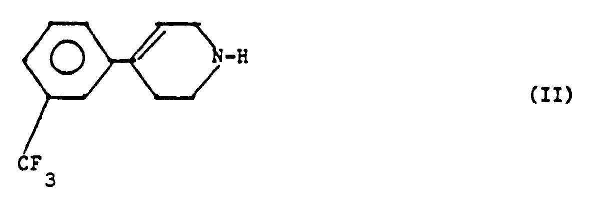
- soit avec un composé de formule (IXa)
X - R - CO - G (IXa)
dans lequel
X est un atome de chlore, brome, iode ou un groupe partant, et
G est tel que défini ci-dessus,
- soit avec un composé de formule (IXb)
X - Q - CO - M (IXb)
dans lequel X et M sont tels que définis ci-dessus,
et que l'on transforme éventuellement le produit obtenu en un de ses sels d'addition.

7. Procédé de préparation d'un composé de formule V 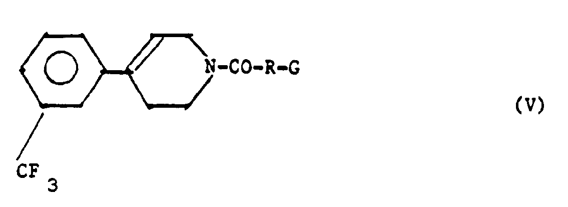 dans laquelle -R- est un radical alkylène à chaine droite ou ramifiée contenant de 1 à 3 atomes de carbone, et -G représente un groupe choisi parmi naphtyle substitué par un groupe hydroxyle ou (C1-C4)alcoxyle; naphtyle substitué par deux groupes hydroxyle, (C1-C4)alcoxyle ou (C1-C4)alkyle; ou naphtyle substitué par un groupe méthylènedioxy, et de ses sels d'addition avec des acides minéraux ou organiques,
caractérisé en ce qu'on fait réagir la 4-(3-trifluorométhylphényl)-1,2,3,6-tétrahydropyridine de formule (II) 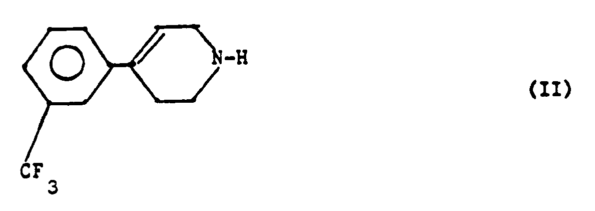 avec un dérivé fonctionnel de l'acide de formule (VIII) 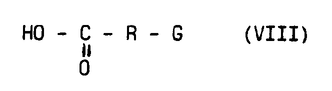 dans laquelle R et G sont tels que définis ci-dessus, et en ce que l'on transforme éventuellement le produit obtenu en un de ses sels d'addition.

8. Procédé pour la préparation d'une composition pharmaceutique, caractérisé en ce que l'on mélange, en tant que principe actif, un composé préparé selon l'une quelconque des revendications 1 à 5, avec un véhicule pharmaceutiquement acceptable.

## Revendications (Revendications pour l'(les) Etat(s) contractant(s) suivant(s): GR)

1. Une 4-(3-trifluorométhylphényl)-1,2,3,6-tétrahydropyridine N-substituée de formule 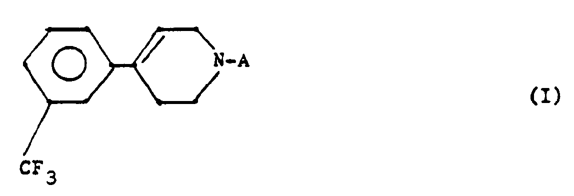 dans laquelle A représente un groupe -E-G ou un groupe -L-M, où
-E- est un radical alkylène à chaine droite ou ramifiée contenant de 2 à 4 atomes de carbone,
-G représente un groupe choisi parmi naphtyle substitué par un groupe hydroxyle ou (C1-C4)alcoxyle; naphtyle substitué par deux groupes hydroxyle, (C1-C4)alcoxyle ou (C1-C4)alkyle; ou naphtyle substitué par un groupe méthylènedioxy,
-L- est un groupe -Q-CH(OH)- dans lequel -Q- est un radical alkylène à chaine droite ou ramifiée contenant de 1 à 3 atomes de carbone, et
-M représente un groupe choisi parmi naphtyle; naphtyle substitué par un ou deux groupes hydroxyle, (C1-C4)alcoxyle ou (C1-C4)alkyle ou par un groupe méthylènedioxy; pyridyle ou pyridyle substitué par un groupe (C1-C4)alkyle,
et leurs sels d'acides minéraux ou organiques.

2. Une 4-(3-trifluorométhylphényl)-1,2,3,6-tétrahydropyridine de la revendication 1 caractérisée en ce que A est un groupe -E-G où -E- est un groupe -CH₂-CH₂- dans lequel des atomes d'hydrogène peut être remplacé par un groupe méthyle et -G est tel que défini dans la revendication 1.

3. Une 4-(3-trifluorométhylphényl)-1,2,3,6-tétrahydropyridine de la revendication 2 caractérisée en ce que -G représente un groupe naphtyle substitué par un groupe hydroxy ou méthoxy ou par deux groupes hydroxy, méthoxy ou méthyle.

4. Une 4-(3-trifluorométhylphényl)-1,2,3,6-tétrahydropyridine de la revendication 1 caractérisée en ce que A est un groupe -L-M où -L- représente un groupe -Q-CH(OH)- dans lequel -Q- est -CH₂- ou -CH(CH₃)- et -M est tel que défini dans la revendication 1.

5. Une 4-(3-trifluorométhylphényl)-1,2,3,6-tétrahydropyridine de la revendication 4 caractérisée en ce que -M représente un groupe naphtyle; naphtyle substitué par un groupe ou deux groupes hydroxy, méthoxy, méthyle; ou un groupe pyridyle.

6. Un procédé de préparation d'un composé de formule (I) 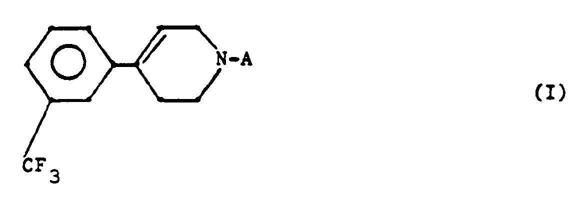 dans laquelle A représente un groupe -E-G ou un groupe -L-M, où
-E- est un radical alkylène à chaine droite ou ramifiée contenant de 2 à 4 atomes de carbone,
-G représente un groupe choisi parmi naphtyle substitué par un groupe hydroxyle ou (C1-C4)alcoxyle; naphtyle substitué par deux groupes hydroxyle, (C1-C4)alcoxyle ou (C1-C4)alkyle; ou naphtyle substitué par un groupe méthylènedioxy,
-L est un groupe -Q-CH(OH)- dans lequel -Q- est un radical alkylène à chaine droite ou ramifiée contenant de 1 à 3 atomes de carbone et
-M représente un groupe choisi parmi naphtyle; naphtyle substitué par un ou deux groupes hydroxyle, (C1-C4)alcoxyle ou (C1-C4)alkyle ou par un groupe méthylènedioxy; pyridyle ou pyridyle substitué par un groupe (C1-C4)alkyle,
ou d'un de ses sels d'addition avec des acides minéraux ou organiques, caractérisé en ce que:
(a) on fait réagir la 4-(3-trifluorométhylphényl)-1,2,3,6-tétrahydropyridine (II) 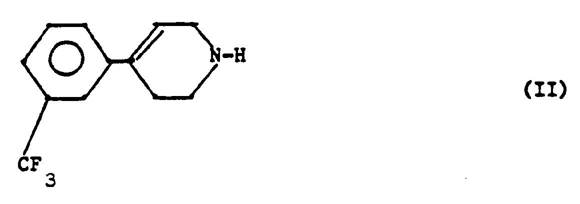 avec un composé de formule (III)
X-A (III)
dans laquelle A a la même signification donnée ci-dessus et X représente un atome de chlore, brome, iode, ou un groupe partant ; ou
(b) lorsqu'on veut obtenir un composé de formule (I) dans laquelle A représente un groupe -E-G ou -L-M où -E- est un groupe -R-CH₂-, dans lequel -R- est un radical alkylène à chaine droite ou ramifiée contenant de 1 à 3 atomes de carbone et G, L et M sont tels que défini ci-dessus, on réduit convenablement un composé de formule (IV) 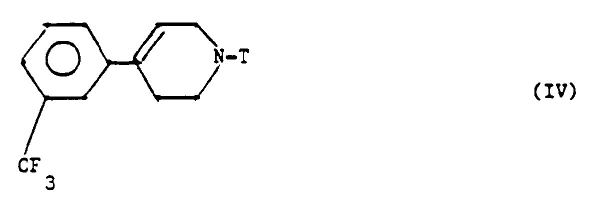 dans laquelle T est un groupe -R-CO-G ou -Q-CO-M où -R-, -Q-, G et M sont tels que définis ci-dessus; ou
(c) lorsqu'on veut obtenir un composé de formule (I) dans laquelle A représente un groupe -E-G où -E- est un groupe -CH₂-R-, -R- et -G étant définis comme ci-dessus, on réduit un composé de formule (V) 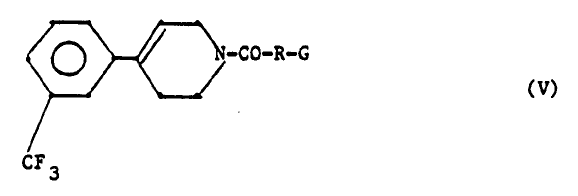 dans laquelle R et C sont tels que définis ci-dessus; ou
(d) lorsqu'on veut obtenir un composé de formule (I) dans laquelle A représente un groupe -L-M où -L- est un groupe -Q-CH(OH)- dans lequel -Q- est -CH₂-, et M est tel que défini ci-dessus, on fait réagir la 4-(3-trifluorométhylphényl)-1,2,3,6-tétrahydropyridine (II) avec un époxyde de formule (VI) 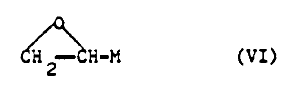 où M est tel que défini ci-dessus;
ce procédé étant ultérieurement caractérisé en ce que,
- lorsqu'on obtient un composé (I) sous forme de base libre, on peut le convertir en un de ses sels d'addition,
- lorsqu'on obtient un composé (I) sous forme de sel d'addition, on peut le convertir en la base libre ou en un autre de ses sels d'addition,
- lorsqu'on obtient un composé de formule (I) dans laquelle A représente un groupe -E-G ou -L-M dans lequel C ou M représentent un groupe naphtyle substitué par un ou deux groupes méthoxyle, on peut le convertir en- le composé correspondant où -G ou M représentent un groupe naphtyle substitué par un ou deux groupes hydroxyle, par deméthylation.

7. Un composé de formule (IV) 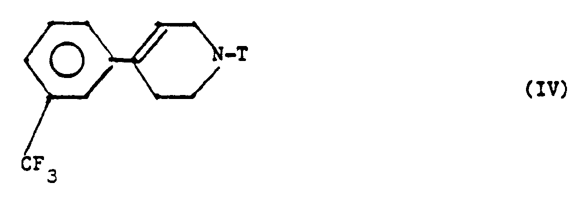 dans laquelle T est un groupe -R-CO-G ou -Q-CO-M où -R- et -Q-, sont un radical alkylène à chaine droite ou ramifiée contenant de 1 à 3 atomes de carbone, -G représente un groupe choisi parmi naphtyle substitué par un groupe hydroxyle ou (C1-C4)alcoxyle; naphtyle substitué par deux groupes hydroxyle, (C -C4)alcoxyle ou (C1-C4)alkyle; ou naphtyle substitué par un groupe méthylènedioxy et -M représente un groupe choisi entre naphtyle; naphtyle substitué par un ou deux groupes hydroxyle, (C1-C4)alcoxyle ou (C1-C4)alkyle ou par un groupe méthylènedioxy; pyridyle; ou pyridyle substitué par un groupe (C1-C4)alkyle et leurs sels d'addition avec des acides mineraux ou organiques.

8. Un composé de formule (V) 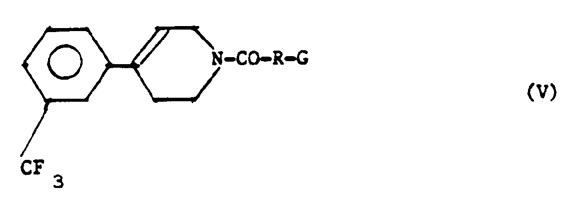 dans laquelle -R- est un radical alkylène à chaine droite ou ramifiée contenant de 1 à 3 atomes de carbone, et -G représente un groupe choisi parmi naphtyle substitué par un groupe hydroxyle ou (C1-C4)alcoxyle; naphtyle substitué par deux groupes hydroxyle, (C1-C4)alcoxyle ou (C1-C4)alkyle; ou naphtyle substitué par un groupe méthylènedioxy.

9. Procédé pour la préparation d'une composition pharmaceutique, caractérisé en ce que l'on mélange, en tant que principe actif, un composé selon l'une quelconque des revendications 1 à 5, avec un véhicule pharmaceutiquement acceptable.

## Patentansprüche (Patentansprüche für folgende(n) Vertragsstaat(en): AT, BE, CH, DE, DK, FR, GB, IT, LI, LU, NL, SE)

1. N-substituiertes 4-(3-Trifluormethylphenyl)-1,2,3,6-tetrahydropyridin der Formel 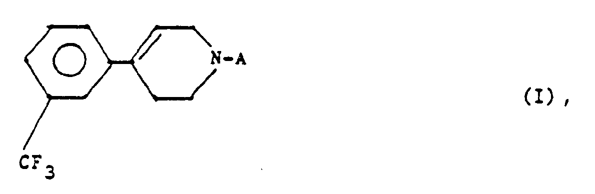 in der A eine Gruppe -E-G oder -L-M darstellt, worin bedeuten:
-E- eine geradkettige oder verzweigte Alkylengruppe mit 2 bis 4 C-Atomen,
-G eine Gruppe, die ausgewählt ist unter Naphthyl, das mit einer Hydroxy- oder einer C₁₋₄-Alkoxy-Gruppe substituiert ist, Naphthyl, das mit zwei Hydroxy-, C₁₋₄-Alkoxy- oder C₁₋₄-Alkylgruppen substituiert ist, oder Naphthyl, das mit einer Methylendioxygruppe substituiert ist,
-L- eine Gruppe -Q-CH(OH)-, worin -Q- eine geradkettige oder verzweigte Alkylengruppe mit 1 bis 3 C-Atomen darstellt, und
-M eine Gruppe, die ausgewählt ist unter Naphthyl, mit ein oder zwei Hydroxy-, C₁₋₄-Alkoxy- oder C₁₋₄-Alkylengruppen substituiertem Naphthyl, mit einer Methylendioxygruppe substituiertem Naphthyl und Pyridyl, das unsubstituiert oder mit einer C₁₋₄-Alkylengruppe substituiert ist,
sowie deren Salze mit anorganischen und organischen Säuren.

2. N-substituiertes 4-(3-Trifluormethylphenyl)-1,2,3,6-tetrahydropyridin nach Anspruch 1, dadurch gekennzeichnet, daß A eine Gruppe -E-G darstellt, worin -E- eine Gruppe -CH₂-CH₂- bedeutet, in der eines der Wasserstoffatome durch eine Methylgruppe ersetzt sein kann und -G wie in Anspruch 1 definiert ist.

3. N-substituiertes 4-(3-Trifluormethylphenyl)-1,2,3,6-tetrahydropyridin nach Anspruch 2, dadurch gekennzeichnet, daß -G eine Naphthylgruppe darstellt, die mit einer Hydroxy- oder Methoxygruppe oder mit zwei Hydroxy-, Methoxy- oder Methylgruppen substituiert ist.

4. N-substituiertes 4-(3-Trifluormethylphenyl)-1,2,3,6-tetrahydropyridin nach Anspruch 1, dadurch gekennzeichnet, daß A eine Gruppe -L-M ist, worin -L- eine Gruppe -Q-CH(OH) darstellt, in der -Q- eine Gruppe -CH₂- oder -CH(CH₃)- ist und -M wie in Anspruch 1 definiert ist.

5. N-substituiertes 4-(3-Trifluormethylphenyl)-1,2,3,6-tetrahydropyridin nach Anspruch 4, dadurch gekennzeichnet, daß -M Naphthyl, mit einer oder zwei Hydroxy-, Methoxy- oder Methylgruppen substituiertes Naphthyl oder Pyridyl darstellt.

6. Verfahren zur Herstellung von Verbindungen der Formel (I), 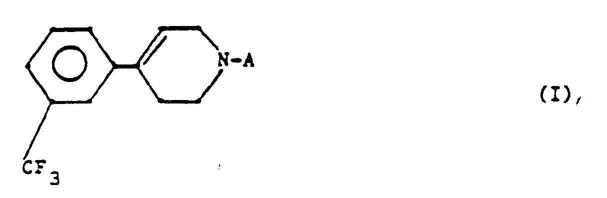 in der A eine Gruppe -E-G oder -L-M darstellt, worin bedeuten:
-E- eine geradkettige oder verzweigte Alkylengruppe mit 2 bis 4 C-Atomen,
-G eine Gruppe, die ausgewählt ist unter
Naphthyl, das mit einer Hydroxy- oder einer C₁₋₄-AlkoxyGruppe substituiert ist,
Naphthyl, das mit zwei Hydroxy-, C₁₋₄-Alkoxy- oder C₁₋₄-Alkylgruppen substituiert ist, und
Naphthyl, das mit einer Methylendioxygruppe substituiert ist,
-L- eine Gruppe -Q-CH(OH)-, worin -Q- eine geradkettige oder verzweigte Alkylengruppe mit 1 bis 3 C-Atomen darstellt und
-M eine Gruppe, die ausgewählt ist unter Naphthyl, mit einer oder zwei Hydroxy-, C₁₋₄-Alkoxy- oder C₁₋₄-Alkylengruppen substituiertem Naphthyl, mit einer Methylendioxygruppe substituiertem Naphthyl und Pyridyl, das unsubstituiert oder mit einer C₁₋₄-Alkylengruppe substituiert ist,
und der Additionssalze mit anorganischen und organischen Säuren, dadurch gekennzeichnet, daß
(a) 4-(3-Trifluormethylphenyl)-1,2,3,6-tetrahydropyridin (II) 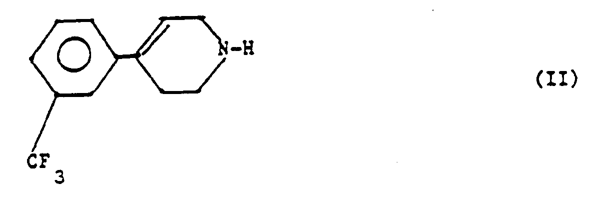 mit einer Verbindung der Formel (III)
X-A (III),
zur Reaktion gebracht wird, worin A dasselbe wie oben bedeutet und X ein Chlor-, Brom- oder Jodatom oder eine Abgangsgruppe ist, oder
(b) wenn eine Verbindung der Formel (I) hergestellt werden soll, in der A eine Gruppe -E-G oder L-M darstellt, worin -E- eine Gruppe -R-CH₂- ist, in der -R- geradkettiges oder verzweigtes C₁₋₃-Alkylen darstellt und G, L und M wie oben definiert sind, eine Verbindung der Formel (IV) 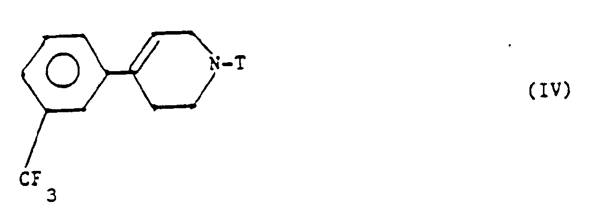 in geeigneter Weise reduziert wird, worin T eine Gruppe R-CO-G oder -Q-CO-M darstellt, in der -R-, -Q-, G und M wie oben definiert sind, oder
(c) wenn eine Verbindung der Formel (I) hergestellt werden soll, worin A eine Gruppe -E-G darstellt, in der -E- eine Gruppe -CH₂-R- ist und -R- und -G wie oben definiert sind, eine Verbindung der Formel (V) 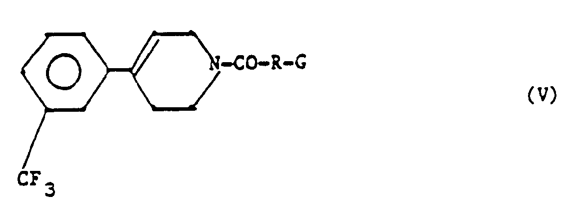 reduziert wird, in der R und G wie oben definiert sind, oder
(d) wenn eine Verbindung der Formel (I) hergestellt werden soll, worin A eine Gruppe -L-M darstellt, in der L eine Gruppe -Q-CH(OH) ist, in der Q -CH₂- bedeutet und M wie oben definiert ist, 1,2,3,6-Tetrahydropyridin (II) mit einem Epoxid der Formel (VI) 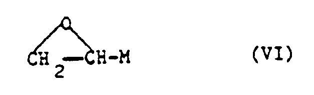 zur Reaktion gebracht wird,
worin M dasselbe wie oben bedeutet, wobei dieses Verfahren weiterhin dadurch gekennzeichnet ist, daß
- in Form der freien Base erhaltene Verbindungen (I) in eines ihrer Additionssalze umgewandelt werden können,
- in Form von Additionssalzen erhaltene Verbindungen (I) in die freie Base oder in ein anderes Additionssalz umgewandelt werden können,
- wenn eine Verbindung der Formel (I) erhalten wird, in der A eine Gruppe -E-G- oder -L-M- darstellt, worin G oder M mit ein oder zwei Methoxygruppen substituiertes Naphthyl ist, diese durch Demethylierung in die entsprechende Verbindung umgewandelt werden kann, in der G oder M mit einer oder zwei Hydroxygruppen substituiertes Naphthyl ist.

7. Verbindungen der Formel (IV) 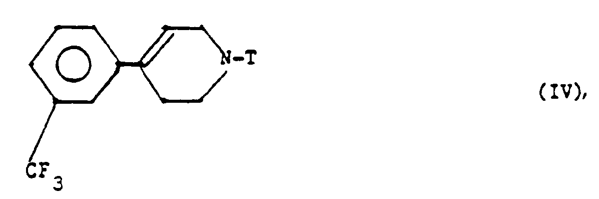 in der bedeuten:
- T eine Gruppe -R-CO-G oder -Q-CO-M, in der -R- und -Q- geradkettiges oder verzweigtes C₁₋₃-Alkyl darstellen,
-G eine Gruppe, die ausgewählt ist unter Naphthyl, das mit einer Hydroxy- oder einer C₁₋₄-Alkoxygruppe substituiert ist,
Naphthyl, das mit zwei Hydroxy-, C₁₋₄-Alkoxy- oder C₁₋₄-Alkylgruppen substituiert ist, und Naphthyl, das mit einer Methylendioxygruppe substituiert ist,
-M eine Gruppe, die ausgewählt ist unter Naphthyl, mit ein oder zwei Hydroxy-, C₁₋₄-Alkoxy- oder C₁₋₄-Alkylengruppen substituiertem Naphthyl, mit einer Methylendioxygruppe substituiertem Naphthyl und Pyridyl, das unsubstituiert oder mit einer C₁₋₄-Alkylengruppe substituiert ist
sowie deren Additionssalze mit anorganischen und organischen Säuren.

8. Verbindungen der Formel (V) 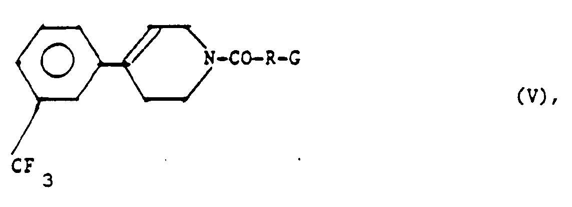 in der bedeuten:
-R- geradkettiges oder verzeigtes C₁₋₃-Alkyl und
-G eine Gruppe, die ausgewählt ist unter Naphthyl, das mit einer Hydroxy- oder einer C₁₋₄-Alkoxygruppe substituiert ist,
Naphthyl, das mit zwei Hydroxy-, C₁₋₄-Alkoxy- oder C₁₋₄-Alkylgruppen substituiert ist, und
Naphthyl, das mit einer Methylendioxygruppe substituiert ist.

9. Pharmazeutische Zusammensetzungen, dadurch gekennzeichnet, daß sie als Wirkstoff mindestens eine Verbindung der Formel (I) 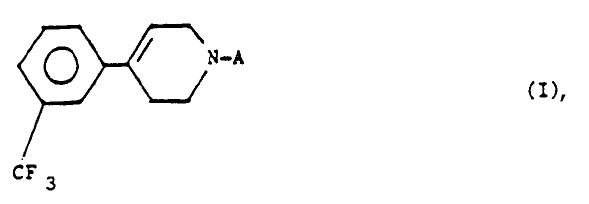 in der A eine Gruppe -E-G oder -L-M darstellt, worin bedeuten:
-E- eine geradkettige oder verzweigte Alkylengruppe mit 2 bis 4 C-Atomen,
-G eine Gruppe, die ausgewählt ist unter Naphthyl, das mit einer Hydroxy- oder einer C₁₋₄-Alkoxygruppe substituiert ist, Naphthyl, das mit zwei Hydroxy-, C₁₋₄-Alkoxy- oder C₁₋₄-Alkylgruppen substituiert ist, und Naphthyl, das mit einer Methylendioxygruppe substituiert ist,
-L- eine Gruppe -Q-CH(OH)-, worin -Q- eine geradkettige oder verzweigte Alkylengruppe mit 1 bis 3 C-Atomen darstellt, und
-M eine Gruppe, die ausgewählt ist unter Naphthyl, mit einer oder zwei Hydroxy-, C₁₋₄-Alkoxy- oder C₁₋₄-Alkylengruppen substituiertem Naphthyl, mit einer Methylendioxygruppe substituiertem Naphthyl und Pyridyl, das unsubstituiert oder mit einer C₁₋₄-Alkylengruppe substituiert ist,
oder eines ihrer pharmazeutisch akzeptablen Additionssalze mit einem Vehikel und/oder pharmazeutisch akzeptablen Excipientien enthalten.

10. Pharmazeutische Zusammensetzungen nach Anspruch 9 zur Verwendung bei der Behandlung von Störungen der Darmmotilität.

## Patentansprüche (Patentansprüche für folgende(n) Vertragsstaat(en): ES)

1. Verfahren zur Herstellung von Verbindungen der Formel (I), 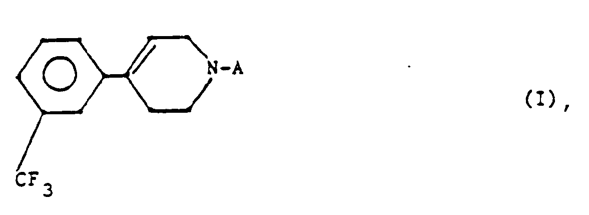 in der A eine Gruppe -E-G oder -L-M darstellt, worin bedeuten:
-E- eine geradkettige oder verzweigte Alkylengruppe mit 2 bis 4 C-Atomen,
-G eine Gruppe, die ausgewählt ist unter
Naphthyl, das mit einer Hydroxy- oder einer C₁₋₄-Alkoxy- Gruppe substituiert ist,
Naphthyl, das mit zwei Hydroxy-, C₁₋₄-Alkoxy- oder C₁₋₄-Alkylgruppen substituiert ist, Naphthyl, das mit einer Methylendioxygruppe substituiert ist,
-L- eine Gruppe -Q-CH(OH)-, worin -Q- eine geradkettige oder verzweigte Alkylengruppe mit 1 bis 3 C-Atomen darstellt und
-M eine Gruppe, die ausgewählt ist unter Naphthyl, mit ein oder zwei Hydroxy-, C1₋₄-Alkoxy- oder C₁₋₄-Alkylengruppen substituiertem Naphthyl, mit einer Methylendioxygruppe substituiertem Naphthyl und Pyridyl, das unsubstituiert oder mit einer C₁₋₄-Alkylengruppe substituiert ist,
und der Additionssalze mit anorganischen und organischen Säuren, dadurch gekennzeichnet, daß
(a) 4-(3-Trifluormethylphenyl)-1,2,3,6-tetrahydropyridin (II) 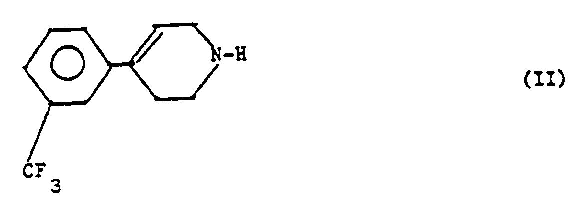 mit einer Verbindung der Formel (III)
X-A (III),
zur Reaktion gebracht wird, worin A dasselbe wie oben bedeutet und X ein Chlor-, Brom- oder Jodatom oder eine Abgangsgruppe ist, oder
(b) wenn eine Verbindung der Formel (I) hergestellt werden soll, in der A eine Gruppe -E-G oder L-M darstellt, worin -E- eine Gruppe -R-CH₂- ist, und in der -R- geradkettiges oder verzweigtes C₁₋₃-Alkylen darstellt und G, L und M wie oben definiert sind, eine Verbindung der Formel (IV) 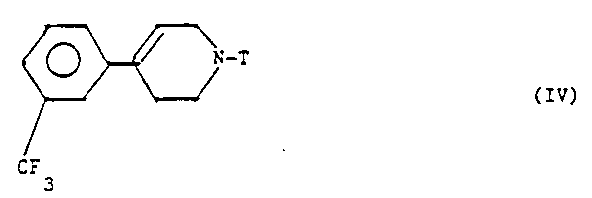 in geeigneter Weise reduziert wird, worin T eine Gruppe -R-CO-G oder -Q-CO-M darstellt, in der -R-, -Q-, G und M wie oben definiert sind, oder
(c) wenn eine Verbindung der Formel (I) hergestellt werden soll, worin A eine Gruppe -E-G darstellt, in der -E- eine Gruppe -CH₂ R- ist und -R- und -G wie oben definiert sind, eine Verbindung der Formel (V) 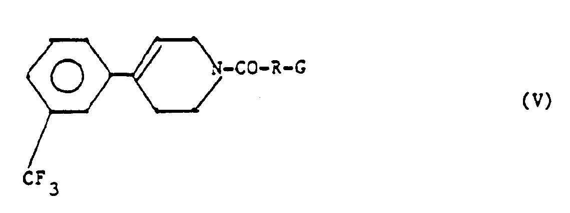 reduziert wird, in der R und G wie oben definiert sind, oder
(d) wenn eine Verbindung der Formel (I) hergestellt werden soll, worin A eine Gruppe -L-M darstellt, in der L eine Gruppe -Q-CH(OH)- ist, in der Q -CH₂- bedeutet, und M wie oben definiert ist, 1,2,3,6-Tetrahydropyridin (II) mit einem Epoxid der Formel (VI) 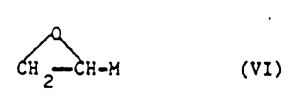 zur Reaktion gebracht wird,
worin M dasselbe wie oben bedeutet, wobei dieses Verfahren weiterhin dadurch gekennzeichnet ist, daß in Form der freien Base erhaltene Verbindungen (I) in eines ihrer Additionssalze umgewandelt werden können,
- in Form von Additionssalzen erhaltene Verbindungen (I) in die freie Base oder in ein anderes Additionssalz umgewandelt werden können,
- wenn eine Verbindung der Formel (I) erhalten wird, in der A eine Gruppe -E-G- oder -L-M- darstellt, worin G oder M mit einer oder zwei Methoxygruppen substituiertes Naphthyl ist, diese durch Demethylierung in die entsprechende Verbindung umgewandelt werden kann, in der -G oder M mit ein oder zwei Hydroxygruppen substituiertes Naphthyl ist.

2. Verfahren nach Anspruch 1 zur Herstellung eines 4-(3-Trifluormethylphenyl)-1,2,3,6-tetrahydropyridins der Formel (I),
worin A eine Gruppe -E-G darstellt, in der -E- eine Gruppe CH₂-CH₂- ist, wobei eines der Wasserstoffatome durch Methyl ersetzt sein kann, und -G wie in Anspruch 1 definiert ist.

3. Verfahren nach Anspruch 1 zur Herstellung eines 4-(3-Trifluormethylphenyl)-1,2,3,6-tetrahydropyridins der Formel (I),
worin A eine Gruppe -E-G darstellt, in der -E- eine Gruppe CH₂-CH₂- ist, wobei eines der Wasserstoffatome durch Methyl ersetzt sein kann, und -G ein mit einer Hydroxy- oder Methoxygruppe oder zwei Hydroxy-, Methoxy- oder Methylgruppen substituiertes Naphthyl darstellt.

4. Verfahren nach Anspruch 1 zur Herstellung eines 4-(3-Trifluormethylphenyl)-1,2,3,6-tetrahydropyridins der Formel (I), worin A eine Gruppe -L-M darstellt, in der -L- eine Gruppe -Q-CH(OH)- ist, wobei -Q- -CH₂- oder -CH(CH₃)- darstellt, und -M wie in Anspruch 1 definiert ist.

5. Verfahren nach Anspruch 1 zur Herstellung eines 4-(3-Trifluormethylphenyl)-1,2,3,6-tetrahydropyridins der Formel (I), worin A eine Gruppe -L-M darstellt, in der -L- eine Gruppe -Q-CH(OH)- ist, wobei -Q- -CH₂- oder -CH(CH₃)- darstellt und -M Naphthyl, mit einer oder zwei Hydroxy-, Methoxy- oder Methylgruppen substituiertes Naphthyl oder Pyridyl ist.

6. Verfahren zur Herstellung von Verbindungen der Formel (IV) 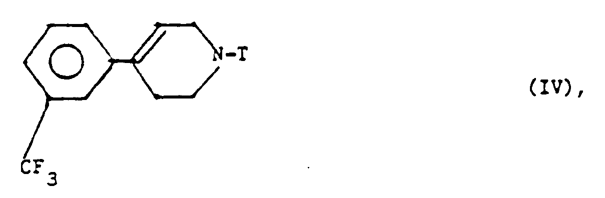 in der bedeuten:
- T eine Gruppe -R-CO-G oder -Q-CO-M, in der -R- und -Q- ein geradkettiges oder verzweigtes C₁₋₃-Alkylen ist,
-G eine Gruppe, die ausgewählt ist unter Naphthyl, das mit einer Hydroxy- oder einer C₁₋₄-Alkoxygruppe substituiert ist, Naphthyl, das mit zwei Hydroxy-, C₁₋₄-Alkoxy- oder C₁₋₄-Alkylgruppen substituiert ist, Naphthyl, das mit einer Methylendioxygruppe substituiert ist,
-M eine Gruppe, die ausgewählt ist unter Naphthyl, mit einer oder zwei Hydroxy-, C₁₋₄-Alkoxy- oder C₁₋₄-Alkylengruppen substituiertem Naphthyl, mit einer Methylendioxygruppe substituiertem Naphthyl und Pyridyl, das unsubstituiert oder mit einer C₁₋₄-Alkylengruppe substituiert ist,
sowie deren Additionssalzen mit anorganischen und organischen Säuren,
dadurch gekennzeichnet, daß das
4-(3-trifluormethylphenyl)-1,2,3,6-tetrahydropyridin der Formel (II) 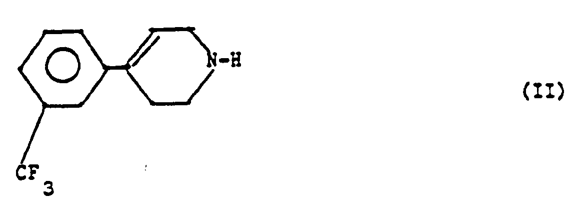
- entweder mit einer Verbindung der Formel (IXa),
X - R - CO - G (IXa),
worin X ein Chlor-, Brom- oder Jodatom oder eine Abgangsgruppe darstellt und
G dasselbe wie oben bedeutet,
- oder mit einer Verbindung der Formel (IXb),
X-Q-CO-M (IXb)
in der X und M wie oben definiert sind,
zur Reaktion gebracht wird und das erhaltene Produkt in eines seiner Additionssalze übergeführt wird.

7. Verfahren zur Herstellung von Verbindungen der Formel V 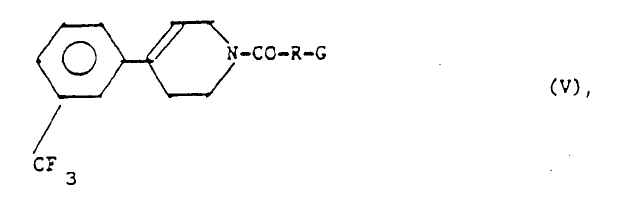 in der bedeuten:
-R- geradkettiges oder verzweigtes C₁₋₃-Alkylen und
-G- eine Gruppe, die ausgewählt ist unter mit einer Hydroxy- oder einer C1₋₄-Alkoxygruppe substituiertem Naphthyl, mit zwei Hydroxy-, C₁₋₄-Alkoxy- oder C₁₋₄-Alkylgruppen substituiertem Naphthyl und mit einer Methylendioxygruppe substituiertem Naphthyl,
sowie ihrer Additionssalze mit anorganischen und organischen Säuren,
dadurch gekennzeichnet, daß das 4-(3-Trifluormethylphenyl)-1,2,3,6-tetrahydropyridin der Formel (II) 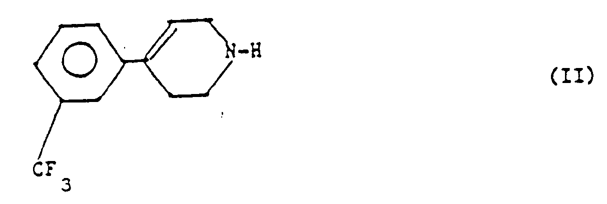 mit einem funktionalen Derivat der Säure der Formel (VIII), 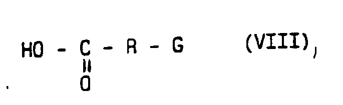 in der R und G dasselbe wie oben bedeuten, zur Reaktion gebracht wird
und das erhaltene Produkt ggf. in eines seiner Additionssalze übergeführt wird.

8. Verfahren zur Herstellung pharmazeutischer Zusammensetzungen, dadurch gekennzeichnet, daß eine nach einem der Ansprüche 1 bis 5 hergestellte Verbindung als Wirkstoff mit einem pharmazeutisch akzeptablen Vehikel gemischt wird.

## Patentansprüche (Patentansprüche für folgende(n) Vertragsstaat(en): ES)

1. N-substituiertes 4-(3-Trifluormethylphenyl)-1,2,3,6-tetrahydropyridin der Formel 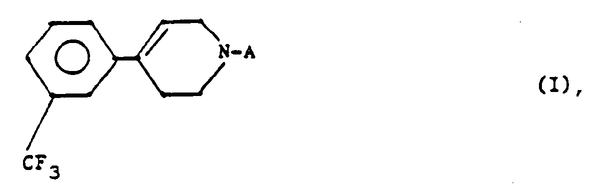 in der A eine Gruppe -E-G oder -L-M darstellt, worin bedeuten:
-E- eine geradkettige oder verzweigte Alkylengruppe mit 2 bis 4 C-Atomen,
-G eine Gruppe, die ausgewählt ist unter
Naphthyl, das mit einer Hydroxy- oder einer C₁₋₄-Alkoxy- Gruppe substituiert ist,
Naphthyl, das mit zwei Hydroxy-, C₁₋₄-Alkoxy- oder C₁₋₄-Alkylgruppen substituiert ist,
Naphthyl, das mit einer Methylendioxygruppe substituiert ist,
-L- eine Gruppe -Q-CH(OH)-, worin -Q- eine geradkettige oder verzweigte Alkylengruppe mit 1 bis 3 C-Atomen darstellt, und
-M eine Gruppe, die ausgewählt ist unter Naphthyl, mit einer oder zwei Hydroxy-, C₁₋₄-Alkoxy- oder C₁₋₄-Alkylengruppen substituiertem Naphthyl, mit einer Methylendioxygruppe substituiertem Naphthyl und Pyridyl, das unsubstituiert oder mit einer C₁₋₄-Alkylengruppe substituiert ist,
sowie deren Additionssalze mit anorganischen und organischen Säuren.

2. N-substituiertes 4-(3-Trifluormethylphenyl)-1,2,3,6-tetrahydropyridin nach Anspruch 1, dadurch gekennzeichnet, daß A eine Gruppe -E-G darstellt, worin -E- eine Gruppe -CH₂-CH₂- bedeutet, in der eines der Wasserstoffatome durch eine Methylgruppe ersetzt sein kann und -G wie in Anspruch 1 definiert ist.

3. N-substituiertes 4-(3-Trifluormethylphenyl)-1,2,3,6-tetrahydropyridin nach Anspruch 2, dadurch gekennzeichnet, daß -G eine Naphthylgruppe darstellt, die mit einer Hydroxy- oder Methoxygruppe oder mit zwei Hydroxy-, Methoxy- oder Methylgruppen substituiert ist.

4. N-substituiertes 4-(3-Trifluormethylphenyl)-1,2,3,6-tetrahydropyridin nach Anspruch 1, dadurch gekennzeichnet, daß A eine Gruppe -L-M ist, worin -L- eine Gruppe -Q-CH(OH)- darstellt, in der -Q- eine Gruppe -CH₂- oder -CH(CH₃)- ist und -M wie in Anspruch 1 definiert ist.

5. N-substituiertes 4-(3-Trifluormethylphenyl)-1,2,3,6-tetrahydropyrin nach Anspruch 4, dadurch gekennzeichnet, daß -M Naphthyl, mit einer oder zwei Hydroxy-, Methoxy- oder Methylgruppen substituiertes Naphthyl oder Pyridyl darstellt.

6. Verfahren zur Herstellung von Verbindungen der Formel (I), 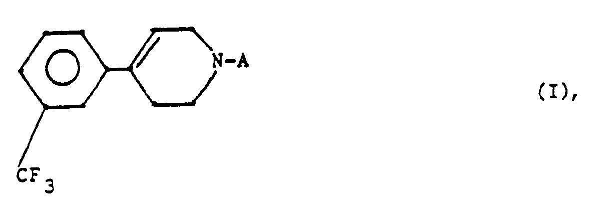 in der A eine Gruppe -E-G oder -L-M darstellt, worin bedeuten:
-E- eine geradkettige oder verzweigte Alkylengruppe mit 2 bis 4 C-Atomen,
-G eine Gruppe, die ausgewählt ist unter
Naphthyl, das mit einer Hydroxy- oder einer C₁₋₄-Alkoxy-Gruppe substituiert ist,
Naphthyl, das mit zwei Hydroxy-, C₁₋₄-Alkoxy- oder C₁₋₄-Alkylgruppen substituiert ist, und
Naphthyl, das mit einer Methylendioxygruppe substituiert ist,
-L- eine Gruppe -Q-CH(OH)-, worin -Q- eine geradkettige oder verzweigte Alkylengruppe mit 1 bis 3 C-Atomen darstellt, und
-M eine Gruppe, die ausgewählt ist unter Naphthyl, mit einer oder zwei Hydroxy-, C₁₋₄-Alkoxy- oder C₁₋₄-Alkylengruppen substituiertem Naphthyl, mit einer Methylendioxygruppe substituiertem Naphthyl und Pyridyl, das unsubstituiert oder mit einer C₁₋₄-Alkylengruppe substituiert ist,
und der Additionssalze mit anorganischen und organischen Säuren, dadurch gekennzeichnet, daß
(a) 4-(3-Trifluormethylphenyl)-1,2,3,6-tetrahydropyridin (II) 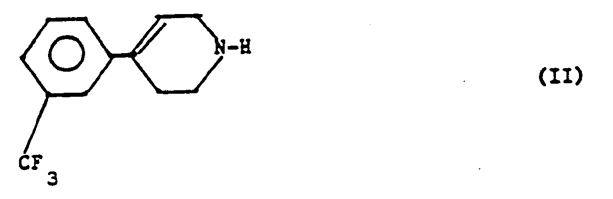 mit einer Verbindung der Formel (III),
X-A (III),
zur Reaktion gebracht wird, worin A dasselbe wie oben bedeutet und X ein Chlor-, Brom- oder Jodatom oder eine Abgangsgruppe ist, oder
(b) wenn eine Verbindung der Formel (I) hergestellt werden soll, in der A eine Gruppe -E-G oder -L-M darstellt, worin -E- eine Gruppe -R-CH₂- ist, in der -R- geradkettiges oder verzweigtes C₁₋₃-Alkylen darstellt und G, L und M wie oben definiert sind, eine Verbindung der Formel (IV) 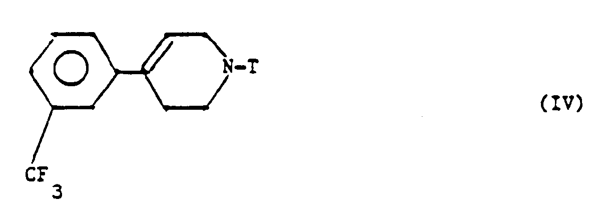 in geeigneter Weise reduziert wird, worin T eine Gruppe R-CO-G oder -Q-CO-M darstellt, in der -R-, -Q-, G und M wie oben definiert sind, oder
(c) wenn eine Verbindung der Formel (I) hergestellt werden soll, worin A eine Gruppe -E-G darstellt, in der -E- eine Gruppe -CH₂-R- ist und -R- und -G wie oben definiert sind, eine Verbindung der Formel (V) 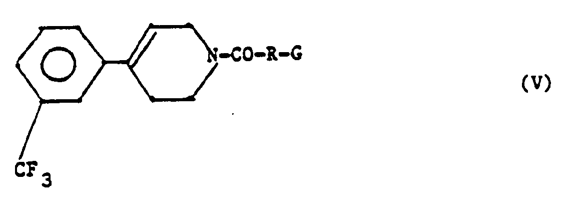 reduziert wird, in der R und G wie oben definiert sind, oder
(d) wenn eine Verbindung der Formel (I) hergestellt werden soll, worin A eine Gruppe -L-M darstellt, in der L eine Gruppe -Q-CH(OH)- ist, in der Q -CH₂- bedeutet und M wie oben definiert ist, 1,2,3,6-Tetrahydropyridin (II) mit einem Epoxid der Formel (VI) 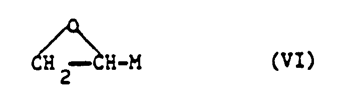 zur Reaktion gebracht wird,
worin M dasselbe wie oben bedeutet, wobei dieses Verfahren weiterhin dadurch gekennzeichnet ist, daß
- in Form der freien Base erhaltene Verbindungen (I) in eines ihrer Additionssalze umgewandelt werden können,
- in Form von Additionssalzen erhaltene Verbindungen (I) in die freie Base oder in ein anderes Additionssalz umgewandelt werden können,
- wenn eine Verbindung der Formel (I) erhalten wird, in der A eine Gruppe -E-G- oder -L-M- darstellt, worin G oder M mit ein oder zwei Methoxygruppen substituiertes Naphthyl ist, diese durch Demethylierung in die entsprechende Verbindung umgewandelt werden kann, in der -G oder M mit ein oder zwei Hydroxygruppen substituiertes Naphthyl ist.

7. Verbindungen der Formel (IV) 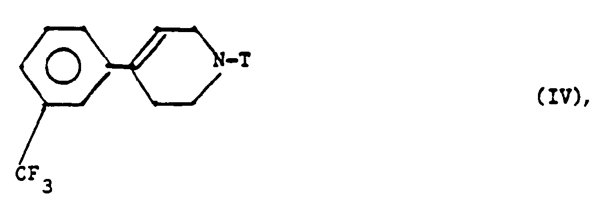 in der bedeuten:
- T eine Gruppe -R-CO-G oder -Q-CO-M, in der -R- und -Q-geradkettiges oder verzweigtes C₁₋₃-Alkyl ist,
-G eine Gruppe, die ausgewählt ist unter
Naphthyl, das mit einer Hydroxy- oder einer C₁₋₄-Alkoxygruppe substituiert ist,
Naphthyl, das mit zwei Hydroxy-, C₁₋₄-Alkoxy- oder C₁₋₄-Alkylgruppen substituiert ist, und
Naphthyl, das mit einer Methylendioxygruppe substituiert ist,
-M eine Gruppe, die ausgewählt ist unter Naphthyl, mit einer oder zwei Hydroxy-, C₁₋₄-Alkoxy- oder C₁₋₄-Alkylengruppen substituiertem Naphthyl, mit einer Methylendioxygruppe substituiertem Naphthyl und Pyridyl, das unsubstituiert oder mit einer C₁₋₄-Alkylengruppe substituiert ist,
sowie deren Additionssalze mit anorganischen und organischen Säuren.

8. Verbindungen der Formel (V) 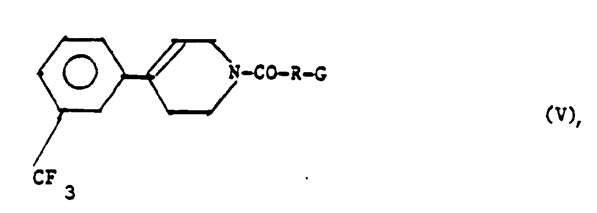 in der bedeuten:
-R- geradkettiges oder verzeigtes C₁₋₃-Alkyl und
-G eine Gruppe, die ausgewählt ist unter
Naphthyl, das mit einer Hydroxy- oder einer C₁₋₄-Alkoxygruppe substituiert ist,
Naphthyl, das mit zwei Hydroxy-, C₁₋₄-Alkoxy- oder C₁₋₄-Alkylgruppen substituiert ist,
Naphthyl, das mit einer Methylendioxygruppe substituiert ist.

9. Verfahren zur Herstellung pharmazeutischer Zusammensetzungen, dadurch gekennzeichnet, daß eine Verbindung nach einem der Ansprüche 1 bis 5 als Wirkstoff mit einem pharmazeutisch akzeptablen Vehikel gemischt wird.

## Claims (Claims for the following Contracting State(s): AT, BE, CH, DE, DK, FR, GB, IT, LI, LU, NL, SE.)

1. A N-substituted 4-(3-trifluoromethylphenyl)-1,2,3,6-tetrahydropyridine of formula 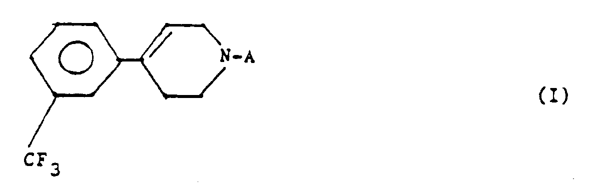 in which A represents a group -E-G or a group -L-M, where
-E- is a straight or branched alkylene radical of from 2 to 4 carbon atoms,
-G represents a group selected from naphthyl substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group,
-L- is a group -Q-CH(OH)- in which -Q- is a straight or branched alkylene radical of from 1 to 3 carbon atoms, and
-M represents a group selected from naphthyl; naphthyl substituted with one or two hydroxy, (C₁-C₄)alkoxy or (C₁-C₄)alkyl groups or with a methylenedioxy group; pyridyl or pyridyl substituted with a (C₁-C₄)alkyl group,
and the salts thereof with mineral or organic acids.

2. A 4-(3-trifluoromethylphenyl)-1,2,3,6-tetrahydropyridine according to claim 1, characterized in that A is a group -E-G where -E- stands for a group -CH₂-CH₂- in which one of the hydrogen atoms may be replaced by a methyl group and -G is as defined in claim 1.

3. A 4-(3-trifluoromethylphenyl)-1,2,3,6-tetrahydropyridine according to claim 2, characterized in that -G represents a naphthyl group substituted with a hydroxy or methoxy group or with two hydroxy, methoxy or methyl groups.

4. A 4-(3-trifluoromethylphenyl)-1,2,3,6-tetrahydropyridine according to claim 1, characterized in that A is a group -L-M where -L- represents a group -Q-CH(OH)- in which -Q- is -CH₂- or -CH(CH₃)- and -M is as defined in claim 1.

5. A 4-(3-trifluoromethylphenyl)-1,2,3,6-tetrahydropyridine according to claim 4, characterized in that -M represents a naphthyl group; naphthyl substituted with one or two hydroxy, methoxy or methyl groups; or a pyridyl group.

6. A process for the preparation of a compound of formula (I) 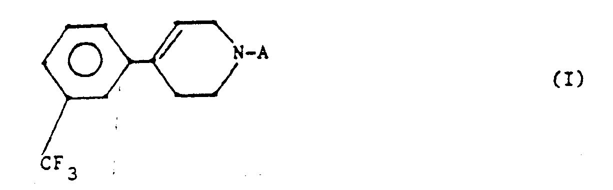 in which A represents a group -E-G or a group -L-M, where
-E- is a straight or branched alkylene radical of from 2 to 4 carbon atoms,
-G represents a group selected from naphthyl substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group,
-L- is a group -Q-CH(OH)- in which -Q- is a straight or branched alkylene radical of from 1 to 3 carbon atoms, and
-M represents a group selected from naphthyl; naphthyl substituted with one or two hydroxy, (C₁-C₄)alkoxy or (C₁-C₄)alkyl groups or with a methylenedioxy group; pyridyl or pyridyl substituted with a (C₁-C₄)alkyl group,
or of one of its addition salts with mineral or organic acids, characterized in that:
(a) 4-(3-trifluoromethylphenyl)-1,2,3,6-tetrahydropyridine (II) 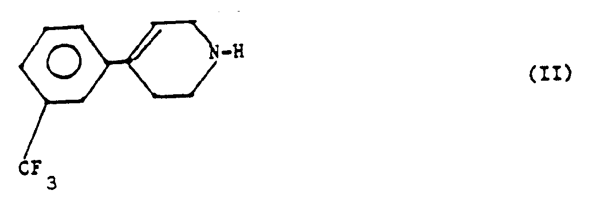 is reacted with a compound of formula (III)
X-A (III)
in which A is as defined above and X represents a chlorine, bromine, iodine atom, or a leaving group; or
(b) when a compound of formula (I) is desired in which A represents a group -E-G or -L-M where -E- is a group -R-CH₂- in which R is a straight or branched alkylene radical of from 1 to 3 carbon atoms and -G, -L-, and -M are as defined above, a compound of formula (IV) 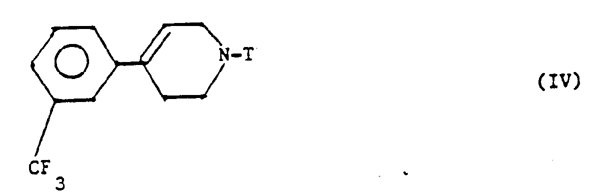 is suitably reduced in which -T stands for a group -R-CO-G or -Q- CO-M where -R-, -Q-, -G, and -M are as defined above; or
(c) when a compound of formula (I) is desired in which A represents a group -E-G where -E- is a group -CH₂-R-, -R- and -G being as defined above, a compound of formula (V) 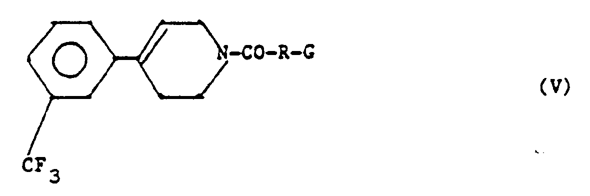 is reduced in which -R- and -G are as defined above; or
(d) when a compound of formula (I) is desired in which A is the group -L-M where -L- is a group -Q-CH(OH)- in which -Q- is -CH₂-, and -M is as defined above, 4-(3-trifluoromethylphenyl)-1,2,3,6-tetrahydropyridine (II) is reacted with the epoxide of formula (VI) 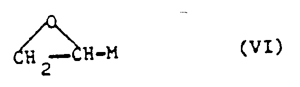 where M is as defined above;
said process being further characterized in that,
- when a compound (I) is obtained as the free base, it may be converted into an addition salt thereof,
- when a compound (I) is obtained as an addition salt, it may be converted into the free base or in another addition salt thereof,
- when a compound of formula (I) is obtained in which A represents a group -E-G or -L-M in which -G or M represent a naphthyl group substituted with one or two methoxy groups, it may be converted into the corresponding compound where G or -M represent a naphthyl group substituted with one or two hydroxy groups by demethylation.

7. A compound of formula (IV) 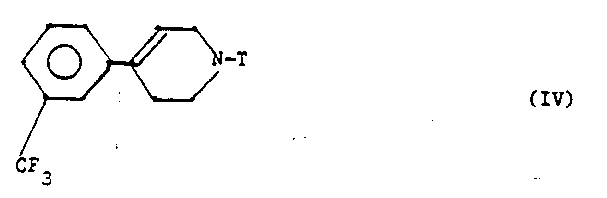 in which -T represents a group -R-CO-G or -Q-CO-M where -R- and -Q-, represent a straight or branched alkylene radical of from 1 to 3 carbon atoms, -G represents a group selected from naphthyl substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group, and -M represents a group selected from naphthyl; naphthyl substituted with one or two hydroxy, (C₁-C₄)alkoxy or (C₁-C₄)alkyl groups or with a methylenedioxy group; pyridyl; or pyridyl substituted with a (C₁-C₄)alkyl group and the addition salts thereof with mineral or organic acids.

8. A compound of formula (V) 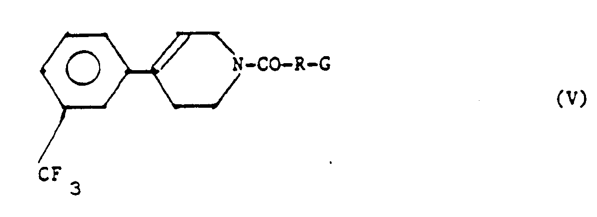 in which -R- represents a straight or branched alkylene radical of from 1 to 3 carbon atoms, and -G represents a group selected from naphthyl substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group.

9. A pharmaceutical composition characterized in that it comprises at least one compound of formula (I) 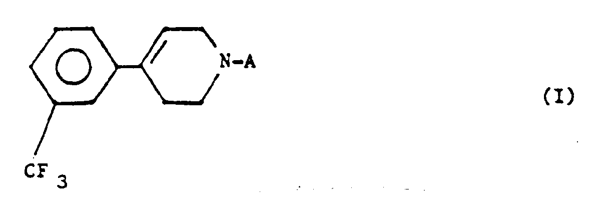 in which A represents a group -E-G or a group -L-M, where
-E- is a straight or branched alkylene radical of from 2 to 4 carbon atoms,
-G represents a group selected from naphthyl substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group,
-L- is a group -Q-CH(OH)- in which -Q- is a straight or branched alkylene radical of from 1 to 3 carbon atoms, and
-M represents a group selected from naphthyl; naphthyl substituted with one or two hydroxy, (C₁-C₄)alkoxy or (C₁-C₄)alkyl groups or with a methylenedioxy group; pyridyl; or pyridyl substituted with a (C₁-C₄)alkyl group,
or one of its pharmaceutically acceptable addition salts, as the active ingredient, together with pharmaceutically acceptable carrier and/or excipients.

10. A pharmaceutical composition according to claim 9 for use in the treatment of intestinal motility disorders.

## Claims (Claims for the following Contracting State(s): ES)

1. Process for preparing a compound of formula (I) 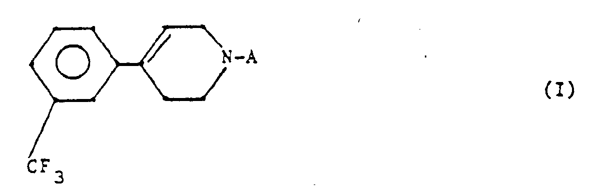 in which A represents a group -E-G or a group -L-M, where
-E- is a straight or branched alkylene radical of from 2 to 4 carbon atoms,
-G represents a group selected from naphthyl substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group,
-L- is a group -Q-CH(OH)- in which -Q- is a straight or branched alkylene radical of from 1 to 3 carbon atoms, and
-M represents a group selected from naphthyl; naphthyl substituted with one or two hydroxy, (C₁-C₄)alkoxy or (C₁-C₄)alkyl groups or with a methylenedioxy group; pyridyl or pyridyl substituted with a (C₁-C₄)alkyl group,
or of one of its addition salts with mineral or organic acids, characterized in that:
(a) 4-(3-trifluoromethylphenyl)-1,2,3,6-tetrahydropyridine (II) 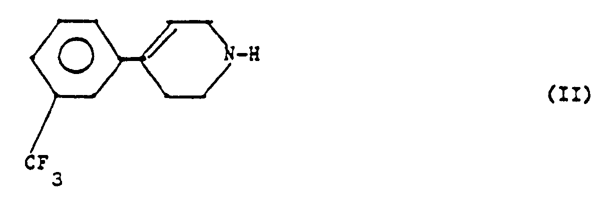 is reacted with a compound of formula (III)
X-A (III)
in which A is as defined above and X represents a chlorine, bromine, iodine atom, or a leaving group; or
(b) when a compound of formula (I) is desired in which A represents a group -E-G or -L-M where -E- is a group -R-CH₂- in which R is a straight or branched alkylene radical of from 1 to 3 carbon atoms and G, -L-, and -M are as defined above, a compound of formula (IV) 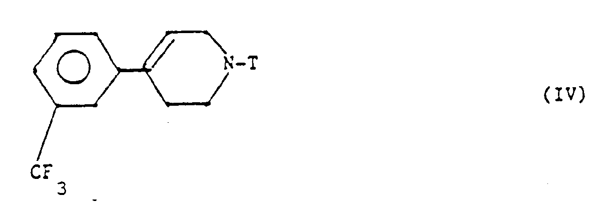 is reduced suitably in which -T stands for a group -R-CO-G or -Q-CO-M where -R-, -Q-, -G, and -M are as defined above; or
(c) when a compound of formula (I) is desired in which A represents a group -E-G where -E- is a group -CH₂-R-, -R- and -G being as defined above, a compound of formula (V) 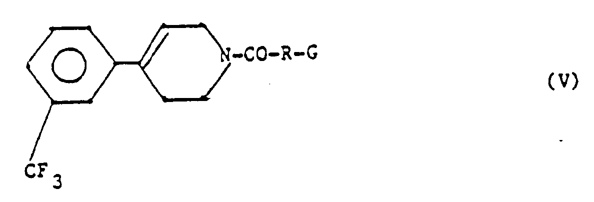 is reduced in which -R- and -G are as defined above; or
(d) when a compound of formula (I) is desired in which A is the group -L-M where -L- is a group -Q-CH(OH)- in which -Q- is -CH₂-, and -M is as defined above, 4-(3-trifluoromethylphenyl)-1,2,3,6-tetrahydropyridine (II) is reacted with the epoxide of formula (VI) 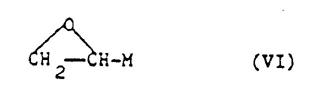 where M is as defined above;
said process being further characterized in that,
- when a compound (I) is obtained as the free base, it may be converted into an addition salt thereof,
- when a compound (I) is obtained as an addition salt, it may be converted into the free base or in another addition salt thereof,
- when a compound of formula (I) is obtained in which A represents a group -E-G or -L-M in which G or M represent a naphthyl group substituted with one or two methoxy groups, it may be converted into the corresponding compound where -G or M represent a naphthyl group substituted with one or two hydroxy groups by demethylation.

2. Process according to claim 1 for the preparation of a 4-(3-trifluoromethylphenyl)-1,2,3,6-tetrahydropyridine of formula (I) in which A is a group -E-G where -E- stands for a group -CH₂-CH₂- where one of the hydrogen atoms may be replaced by a methyl group and -G is as defined in claim 1.

3. Process according to claim 1 for the preparation of a 4-(3-trifluoromethylphenyl)-1,2,3,6-tetrahydropyridine of formula (I) in which A is a group -E-G where -E- stands for a group -CH₂-CH₂- where one of the hydrogen atoms may be replaced by a methyl group and -G represents a naphthyl group substituted with a hydroxy or methoxy group or with two hydroxy, methoxy or methyl groups.

4. Process according to claim 1 for the preparation of a 4-(3-trifluoromethylphenyl)-1 ,2,3,6-tetrahydropyridine of formula (I) in which A is a group -L-M where -L- represents a group -Q-CH(OH)- in which -Q- is -CH₂- or -CH(CH₃)- and -M is as defined in claim 1.

5. Process according to claim 1 for the preparation of a 4-(3-trifluoromethylphenyl)-1,2,3,6-tetrahydropyridine of formula (I) in which A is a group -L-M in which -L- represents a group -Q-CH(OH)- in which -Q- is -CH₂- or -CH(CH₃)- and -M is a naphthyl group; naphtyl substituted with one or two hydroxy, methoxy or methyl; or a pyridyl group.

6. Process for the preparation of a compound of formula (IV) 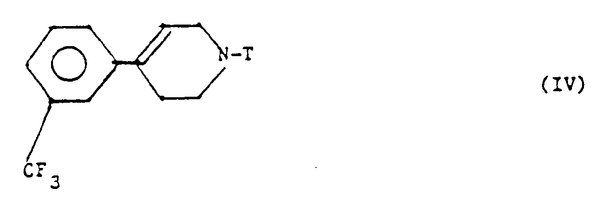 in which -T represents a group -R-CO-G or -Q-CO-M where -R- and -Q-, are a straight or branched alkylene radical of from 1 to 3 carbon atoms, -G represents a group selected from naphthyl substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group, and -M represents a group selected from naphthyl; naphthyl substituted with one or two hydroxy, (C₁-C₄)alkoxy or (C₁-C₄)alkyl groups or with a methylenedioxy group; pyridyl; or pyridyl substituted with a (C₁-C₄)alkyl group and the addition salts thereof with mineral or organic acids,
characterized in that a 4-(3-trifluoromethylphenyl)-1,2,3,6-tetrahydropyridine of formula (II) 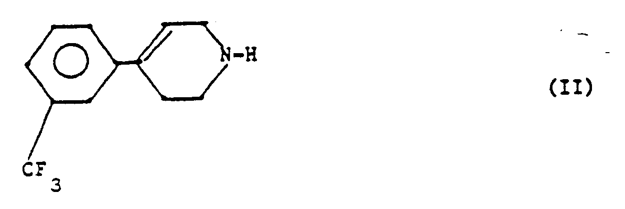
- is reacted either with a compound of formula (IXa)
X - R - CO - G (IXa)
in which
X represents a chlorine, bromine, iodine atom, or a leaving group, and
G is as defined above,
- or with a compound of formula (IXb)
X - Q - CO - M (IXb)
in which X and M are as defined above,
and that the optionally obtained product be converted into one of its addition salts.

7. Process for the preparation of a compound of formula (V) 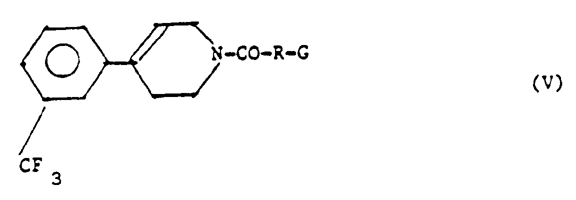 in which -R- is a straight or branched alkylene radical of from 1 to 3 carbon atoms, and -G represents a group selected from naphthyl substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group, and the addition salts thereof with mineral or organic acids,
characterized in that a 4-(3-trifluoromethylphenyl)-1,2,3,6-tetrahydropyridine of formula (II) 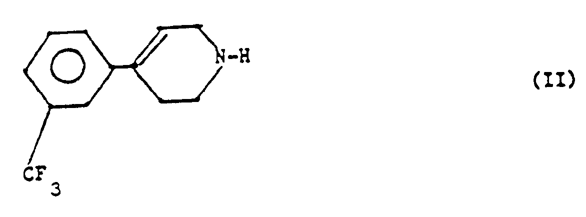 - is reacted with a fonctional derivative of the acid of formula (VIII) 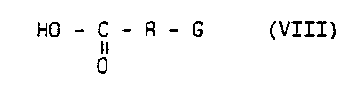 in which R and G are as defined above,
and in that the optionally obtained product is converted into one of its addition salts.

8. Process for the preparation of a pharmaceutical composition, characterized in that a compound prepared according to any one of claims 1 to 5, is mixed, as active principal, with a a pharmaceutically acceptable carrier.

## Claims (Claims for the following Contracting State(s): GR.)

1. A N-substituted 4-(3-trifluoromethylphenyl)-1,2,3,6-tetrahydropyridine of formula 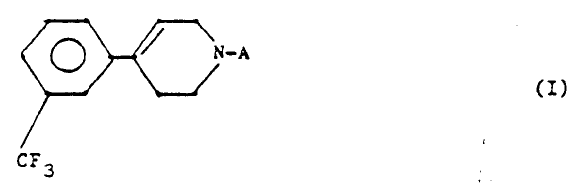 in which A represents a group -E-G or a group -L-M, where
-E- is a straight or branched alkylene radical of from 2 to 4 carbon atoms,
-G represents a group selected from naphthyl substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group,
-L- is a group -Q-CH(OH)- in which -Q- is a straight or branched alkylene radical of from 1 to 3 carbon atoms, and
-M represents a group selected from naphthyl; naphthyl substituted with one or two hydroxy, (C₁-C₄)alkoxy or (C₁-C₄)alkyl groups or with a methylenedioxy group; pyridyl or pyridyl substituted with a (C₁-C₄)alkyl group,
and the salts thereof with mineral or organic acids.

2. A 4-(3-trifluoromethylphenyl)-1,2,3,6-tetrahydropyridine according to claim 1, characterized in that A is a group -E-G where -E- stands for a group -CH₂-CH₂- in which one of the hydrogen atoms may be replaced by a methyl group and -G is as defined in claim 1.

3. A 4-(3-trifluoromethylphenyl)-1,2,3,6-tetrahydropyridine according to claim 2, characterized in that -G represents a naphthyl group substituted with a hydroxy or methoxy group or with two hydroxy, methoxy or methyl groups.

4. A 4-(3-trifluoromethylphenyl)-1,2,3,6-tetrahydropyridine according to claim 1, characterized in that A is a group -L-M where -L- represents a group -Q-CH(OH)- in which -Q- is -CH₂- or -CH(CH₃)- and -M is as defined in claim 1.

5. A 4-(3-trifluoromethylphenyl)-1,2,3,6-tetrahydropyridine according to claim 4, characterized in that -M represents a naphthyl group; naphthyl substituted with one or two hydroxy, methoxy or methyl groups; or a pyridyl group.

6. A process for the preparation of a compound of formula (I) 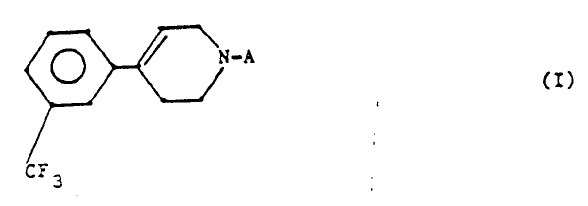 in which A represents a group -E-G or a group -L-M, where
-E- is a straight or branched alkylene radical of from 2 to 4 carbon atoms,
-G represents a group selected from naphthyl substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group,
-L- is a group -Q-CH(OH)- in which -Q- is a straight or branched alkylene radical of from 1 to 3 carbon atoms, and
-M represents a group selected from naphthyl; naphthyl substituted with one or two hydroxy, (C₁-C₄)alkoxy or (C₁-C₄)alkyl groups or with a methylenedioxy group; pyridyl or pyridyl substituted with a (C₁-C₄)alkyl group,
or of one of its addition salts with mineral or organic acids, characterized in that:
(a) 4-(3-trifluoromethylphenyl)-1,2,3,6-tetrahydropyridine (II) 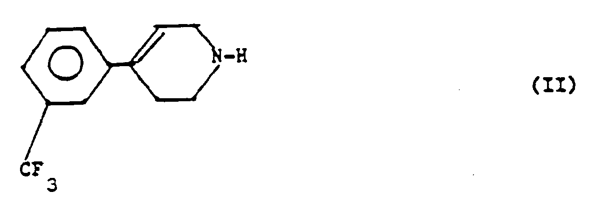 is reacted with a compound of formula (III)
X-A (III)
in which A is as defined above and X represents a chlorine, bromine, iodine atom, or a leaving group; or
(b) when a compound of formula (I) is desired in which A represents a group -E-G or -L-M where -E- is a group -R-CH₂- in which R is a straight or branched alkylene radical of from 1 to 3 carbon atoms and -G, -L-, and -M are as defined above, a compound of formula (IV) 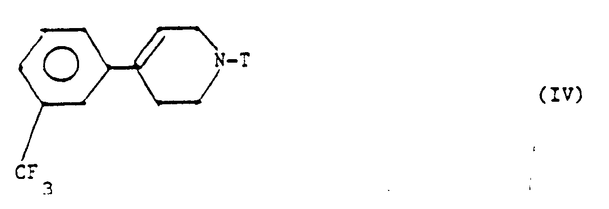 is reduced appropriately in which -T stands for a group -R-CO-G or -Q-CO-M where -R-, -Q-, -G, and -M are as defined above; or
(c) when a compound of formula (I) is desired in which A represents a group -E-G where -E- is a group -CH₂-R-, -R- and -G being as defined above, a compound of formula (V) 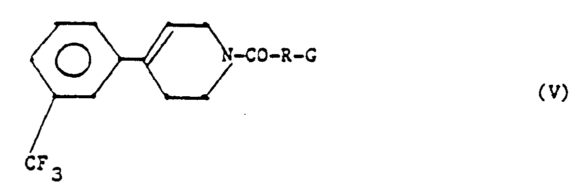 is reduced in which -R- and -G are as defined above; or
(d) when a compound of formula (I) is desired in which A is the group -L-M where -L- is a group -Q-CH(OH)- in which -Q- is -CH₂-, and -M is as defined above, 4-(3-trifluoromethylphenyl)-1,2,3,6-tetrahydropyridine (II) is reacted with the epoxide of formula (VI) 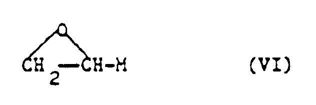 where M is as defined above;
said process being further characterized in that,
- when a compound (I) is obtained as the free base, it may be converted into an addition salt thereof,
- when a compound (I) is obtained as an addition salt, it may be converted into the free base or in another addition salt thereof,
- when a compound of formula (I) is obtained in which A represents a group -E-G or -L-M in which -G or M represent a naphthyl group substituted with one or two methoxy groups, it may be converted into the corresponding compound where G or -M represent a naphthyl group substituted with one or two hydroxy groups by demethylation.

7. A compound of formula (IV) 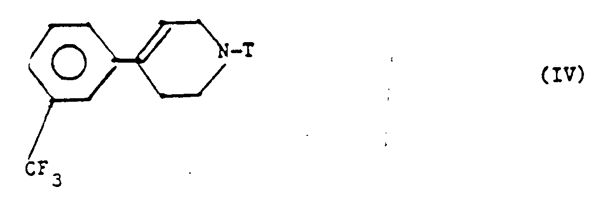 in which -T represents a group -R-CO-G or -Q-CO-M where -R- and -Q-, represent a straight or branched alkylene radical of from 1 to 3 carbon atoms, -G represents a group selected from naphthyl substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group, and -M represents a group selected from naphthyl; naphthyl substituted with one or two hydroxy, (C₁-C₄)alkoxy or (C₁-C₄)alkyl groups or with a methylenedioxy group; pyridyl; or pyridyl substituted with a (C₁-C₄)alkyl group and the addition salts thereof with mineral or organic acids.

8. A compound of formula (V) 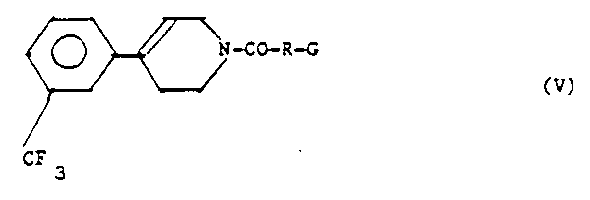 in which -R- represents a straight or branched alkylene radical of from 1 to 3 carbon atoms, and -G represents a naphthyl group substituted with a hydroxy or (C₁-C₄)alkoxy group; naphthyl substituted with two hydroxy, (C₁-C₄)alkoxy or (C₁-C₄)alkyl groups; or naphthyl substituted with a methylenedioxy group.

9. Process for the preparation of a pharmaceutical composition, characterized in that a compound according to any one of claims 1 to 5, is mixed, as active principal, with a a pharmaceutically acceptable carrier.
